(19) Europäisches Patentamt
European Patent Office
Office européen des brevets

(11) EP 4 239 037 A1

(12) **EUROPEAN PATENT APPLICATION**
published in accordance with Art. 153(4) EPC

(43) Date of publication:
**06.09.2023 Bulletin 2023/36**

(21) Application number: **21886349.6**

(22) Date of filing: **28.10.2021**

(51) International Patent Classification (IPC):
***C09K 23/00*** (2022.01) ***C09K 23/22*** (2022.01)
***C09K 23/28*** (2022.01) ***C09K 23/32*** (2022.01)
***A61Q 19/00*** (2006.01) ***A61Q 5/00*** (2006.01)
***A61K 8/04*** (2006.01) ***A61K 8/06*** (2006.01)
***A61K 8/36*** (2006.01) ***A61K 8/42*** (2006.01)
***A61K 8/44*** (2006.01) ***A61K 8/49*** (2006.01)
***A61K 8/64*** (2006.01)

(52) Cooperative Patent Classification (CPC):
**A61K 8/04; A61K 8/06; A61K 8/36; A61K 8/42; A61K 8/44; A61K 8/49; A61K 8/64; A61Q 5/00; A61Q 19/00; C09K 23/00; C09K 23/22; C09K 23/28; C09K 23/32**

(86) International application number:
**PCT/JP2021/039903**

(87) International publication number:
**WO 2022/092232 (05.05.2022 Gazette 2022/18)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **02.11.2020 JP 2020183653**
**18.01.2021 JP 2021005540**
**16.03.2021 JP 2021042818**

(71) Applicant: **Asahi Kasei Finechem Co., Ltd.**
**Osaka-shi, Osaka 530-8205 (JP)**

(72) Inventors:
- **TAMURA, Yoshinaga**
**Tokyo 100-0006 (JP)**
- **TAKASE, Hiroyuki**
**Tokyo 100-0006 (JP)**
- **SEKIGUCHI, Norio**
**Tokyo 100-0006 (JP)**

(74) Representative: **Strehl Schübel-Hopf & Partner**
**Maximilianstrasse 54**
**80538 München (DE)**

# (54) COMPOSITION, METHOD FOR PRODUCING SAME, AND COSMETIC

(57) The present invention aims to provide a composition, particularly a starting material composition for fragrances and cosmetics, which imparts excellent emulsification stability and dispersion stability. The object can be achieved by a composition comprising a component A, and one or more components selected from the group consisting of a component B, a component C, a component D, and a component E (provided that only the component C or only the component D is excluded), as described in the description.



Processed by Luminess, 75001 PARIS (FR)

## Description

### Technical Field

The present invention relates to a composition, particularly a starting material composition for fragrances and cosmetics and a method for producing the same and a cosmetic agent.

### Background Art

Acyl group-containing amino acid derivatives, even when added in a trace amount, as a starting material for fragrances and cosmetics, allows particularly stability, emulsifiability, and dispersibility of weakly acidic formulation having a pH close to that of hair and skin to be satisfactory, and thus have been particularly regarded as important in recent years. For example, Patent Literature 1 discloses a gel-like composition containing an acyl group-containing amino acid derivative, which is a composition having excellent stability and feeling in use. Patent Literature 2 discloses a technique related to contribution of blending of an acyl group-containing amino acid derivative to formulation stability, an aseptic effect, and low irritation in a cosmetic agent.

### Citation List

#### Patent Literature

Patent Literature 1: Japanese Patent No. 4070768
Patent Literature 2: Japanese Patent Laid-Open No. 2006-160686

### Summary of Invention

#### Technical Problem

In formulation for fragrances and cosmetics, in particular, for skin care and hair care applications, emulsifying ability and dispersing ability are highly required. However, conventional formulation for fragrances and cosmetics described in Patent Literatures 1, 2, and the like, it is still difficult to achieve high emulsification stability and dispersion stability.
It is thus an object of the present invention to provide a composition (particularly a starting material composition for fragrances and cosmetics) that imparts excellent emulsification stability and dispersion stability.
In other words, the present inventors have made intensive studies to achieve the object described above and have found that combining an acyl group-containing amino acid derivative having a specific structure with various components enables a composition that imparts excellent emulsification stability and dispersion stability to be provided.
That is to say, the present invention is as follows.

[1] A composition comprising:

a component A shown below; and
one or more components selected from the group consisting of a component B, a component C, a component D, and a component E each shown below, provided that only the component C or only the component D is excluded:

- component A: an acyl group-containing amino acid derivative represented by the following general formula (A) or a salt thereof:

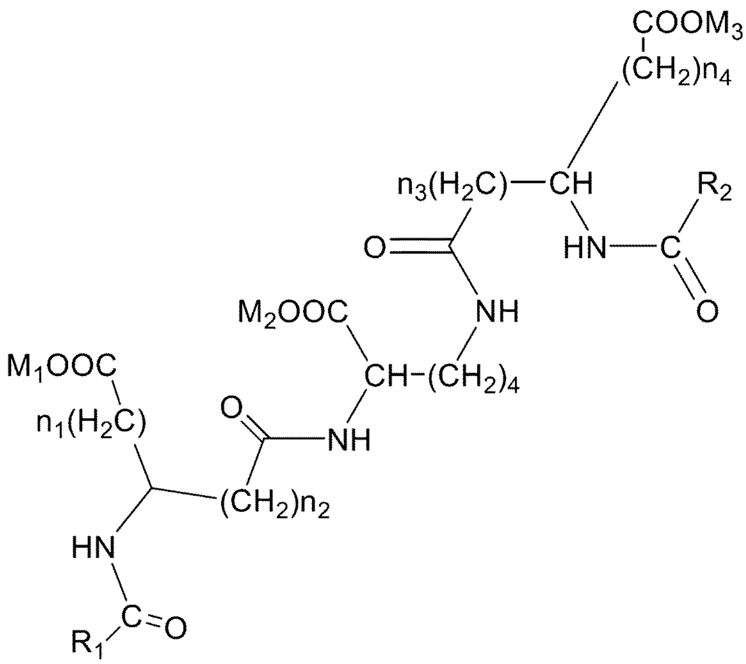

(A)

wherein

R1 and R2 are each independently a saturated or unsaturated linear or branched hydrocarbon group,
M1, M2, and M3 are each independently a hydrogen atom, alkali metal, alkaline earth metal, an organic or inorganic amine, a basic amino acid, choline, aluminum, or zinc,
n1 and n2 are 0 and 2 or 2 and 0, and
n3 and n4 are 0 and 2 or 2 and 0,

- component B: a N-acyl pyrrolidonecarboxylic acid represented by the following general formula (B) or a salt thereof:

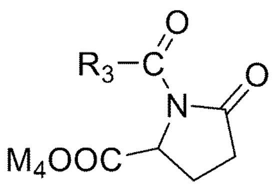

(B)

wherein

R3 is a saturated or unsaturated linear or branched hydrocarbon group, and
M4 is a hydrogen atom, alkali metal, alkaline earth metal, an organic or inorganic amine, a basic amino acid, choline, aluminum, or zinc,

- component C: an acyl glutamic acid represented by the following general formula (C) or salt thereof:

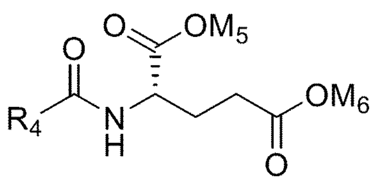

(C)

wherein

R4 is a saturated or unsaturated linear or branched hydrocarbon group, and
M5 and M6 are each independently a hydrogen atom, alkali metal, alkaline earth metal, an organic or inorganic amine, a basic amino acid, choline, aluminum, or zinc,

- component D: a fatty acid represented by the following general formula (D) or salt thereof:

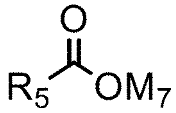
(D)

wherein

R5 is a saturated or unsaturated linear or branched hydrocarbon group, and
M7 is a hydrogen atom, alkali metal, alkaline earth metal, an organic or inorganic amine, a basic amino acid, choline, aluminum, or zinc, and

- component E: a fatty acid amide represented by the following general formula (E):

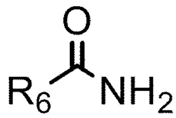
(E)

wherein
R6 is a saturated or unsaturated linear or branched hydrocarbon group.

[2] The composition according to [1], comprising the component A and the component B.
[3] The composition according to [1], comprising the component A and the component E.
[4] The composition according to [1], comprising the component A, the component B, and the component E.
[5] The composition according to any of [1] to [4], wherein R1 to R6 in the general formulas (A) to (E) are each independently a hydrocarbon group having 1 to 29 carbon atoms.
[6] The composition according to any of [1] to [5], wherein M1 to M7 in the general formulas (A) to (D) are each independently Na, K, triethanolamine, Mg, Ca, Al, Zn, arginine, or H.
[7] The composition according to any of [1], [2], [4], [5], and [6], wherein a proportion of the component B with respect to the component A is 0.004% or more.
[8] The composition according to any of [1] and [5] to [7], wherein

an amount of the component C is 0.8% by mass or more based on the total mass of the component A and the component C, and
an amount of the component D is 0.06% by mass or more based on the total mass of the component A and the component D.

[9] The composition according to any of [1] and [3] to [8], wherein an amount of the component E is 0.005% by mass or more based on the mass of the component A.
[10] A method for producing the composition according to any of [1], [2], and [4] to [9], comprising the following steps:

1) reacting glutamic acid or a salt thereof with a fatty acid chloride in a mixed solvent comprising water and an organic solvent to provide a reaction liquid containing a N-acyl glutamic acid or a salt thereof, allowing the reaction liquid to have a pH of 1 to 6 with an acid, and separating the reaction liquid into an organic layer and an aqueous layer at a temperature of 25 to 80°C;
2) concentrating the organic layer to dryness under acidity to convert a portion of the N-acyl glutamic acid or a salt thereof into a N-acyl pyrrolidonecarboxylic acid or a salt thereof;
3) reacting the N-acyl glutamic acid or a salt thereof with an acid anhydride to provide a N-acyl glutamic acid anhydride; and
4) reacting the N-acyl glutamic acid anhydride with lysine or a salt thereof to provide an acyl group-containing

amino acid derivative or a salt thereof.

[11] The production method according to [10], wherein, in the step 2), the organic layer is concentrated to dryness until a loss on drying reaches 10% by mass or less.
[12] The production method according to [10] or [11], wherein the concentration to dryness in the step 2) is performed under reduced pressure at 50°C or more.
[13] The composition according to any of [1] to [9], wherein the composition is a starting material composition for fragrances and cosmetics.
[14] Use of the composition according to any of [1] to [9] as a starting material for fragrances and cosmetics.
[15] A cosmetic agent comprising the composition according to any of [1] to [9].
[16] The cosmetic agent according to [15], having a pH of 4 or more.

Advantageous Effects of Invention

According to the present invention, there can be provided a composition, particularly a starting material composition for fragrances and cosmetics, which imparts excellent emulsification stability and dispersion stability.

Description of Embodiment

Hereinafter, an embodiment for carrying out the present invention (hereinafter simply referred to as "the present embodiment") will now be described in detail. The present embodiment below is an illustration for explaining the present invention and is not intended to limit the present invention to the following contents. The present invention can be carried out after making various modifications within the scope thereof.

[Composition]

The present embodiment relates to a composition, particularly a starting material composition for fragrances and cosmetics, comprising: a component A shown below; and one or more components selected from the group consisting of a component B, a component C, a component D, and a component E each shown below, provided that only the component C or only the component D is excluded, and to a cosmetic comprising the same. Examples of the combination of the components include A + B, A + E, A + B + C, A + B + D, A + B + E, A + C + D, A + C + E, A + B + C + D, A + B + C + E, and A + C + D + E.
The component A is an acyl group-containing amino acid derivative represented by the following general formula (A) or a salt thereof:

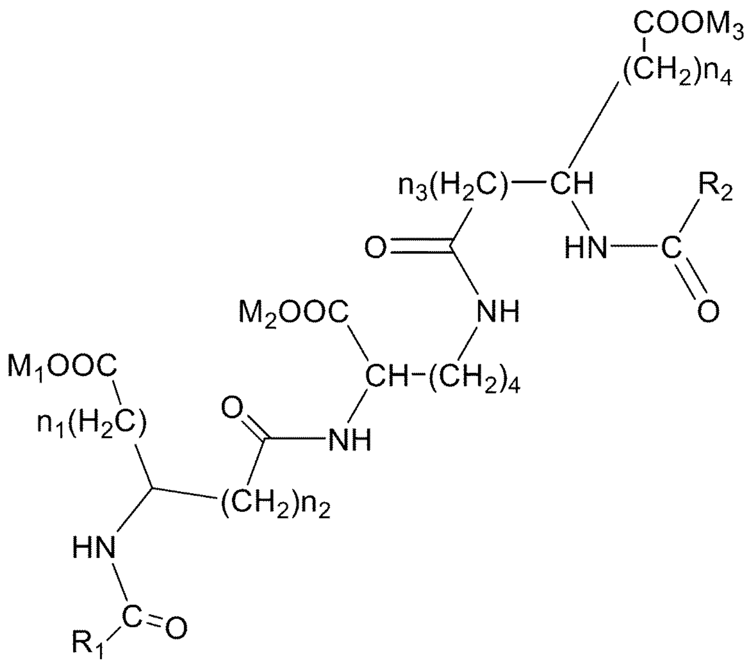

(A)

wherein

R1 and R2 are each independently a saturated or unsaturated linear or branched hydrocarbon group,
M1, M2, and M3 are each independently, a hydrogen atom, alkali metal, alkaline earth metal, an organic or inorganic amine, a basic amino acid, or choline,
n1 and n2 are 0 and 2 or 2 and 0, and
n3 and n4 are 0 and 2 or 2 and 0.

The component B is a N-acyl pyrrolidonecarboxylic acid represented by the following general formula (B) or a salt thereof; and the component B herein is also referred to as an "acyl PCA":

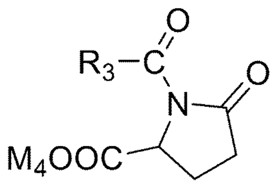
(B)

wherein

R3 is a saturated or unsaturated linear or branched hydrocarbon group, and
M4 is a hydrogen atom, alkali metal, alkaline earth metal, an organic or inorganic amine, a basic amino acid, or choline.

The component C is an acyl glutamic acid represented by the following general formula (C) or a salt thereof:

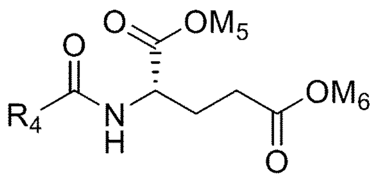
(C)

wherein

R4 is a saturated or unsaturated linear or branched hydrocarbon group, and
M5 and M6 is each independently a hydrogen atom, alkali metal, alkaline earth metal, an organic or inorganic amine, a basic amino acid, or choline.

The component D is a fatty acid represented by the following general formula (D) or a salt thereof:

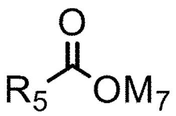
(D)

wherein

R5 is a saturated or unsaturated linear or branched hydrocarbon group, and
M7 is a hydrogen atom, alkali metal, alkaline earth metal, an organic or inorganic amine, a basic amino acid, or choline.

The component E is a fatty acid amide represented by the following general formula (E):

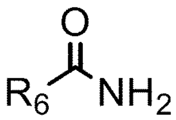 (E)

wherein
R6 is a saturated or unsaturated linear or branched hydrocarbon group.

The starting material composition for fragrances and cosmetics of the present embodiment imparts high initial emulsification stability and dispersion stability to formulation for fragrances and cosmetics and also can impart satisfactory feeling in use thereto.

As R1 and R2 in the general formula (A), any hydrocarbon group can be used irrespective of kinds thereof: saturated, unsaturated, linear, or branched chains. For example, R1 and R2 may be each independently a hydrocarbon group having 9 carbon atoms, a hydrocarbon group having 11 carbon atoms, a hydrocarbon group having 13 carbon atoms, a hydrocarbon group having 15 carbon atoms, a hydrocarbon group having 17 carbon atoms, a hydrocarbon group having 19 carbon atoms, or a hydrocarbon group having 21 carbon atoms. Particularly, as an R1-C(=O)- structure or R2-C(=O)- structure, hydrocarbon groups commonly distributed in the world can be used such as a capryloyl group, an undecylenoyl group, a lauroyl group, a myristoyl group, an isomyristoyl group, a palmitoyl group, an isopalmitoyl group, a stearoyl group, an isostearoyl group, an oleyl group, a linolyl group, a linoleyl group, an arachidyl group, and a behenyl group, and mixed hydrocarbon groups of a composition derived from palm oil, palm kernel oil, or coconut oil also can be used. Examples thereof include hydrocarbon groups having 1 to 29 carbon atoms. The groups are preferably hydrocarbon groups having 7 to 23 carbon atoms. The groups are more preferably hydrocarbon groups having 7 to 17 carbon atoms. Particularly preferably, saturated hydrocarbon groups are used, in which it is not difficult to manage prevention of air oxidation. From the economic viewpoint, the R1-C(=O)-structure and R2-C(=O)- structure are even further preferably a lauroyl group and/or a myristoyl group.

The 3 carboxylic acid groups in the acyl group-containing amino acid derivative represented by the general formula (A) may be all free carboxylic acids, may be all carboxylates, or may be a combination of free carboxylic acid(s) and carboxylate(s).

Examples of carboxylates in the general formula (A) (COOM1, COOM2, COOM3) include, but are not particularly limited to, a single salt or mixture of: an alkali metal salt or alkaline earth metal salt such as a sodium salt, a potassium salt, a lithium salt, a magnesium salt, and a calcium salt; an amine salt such as an ammonium salt, an alkylamine salt, a monoethanolamine salt, a diethanolamine salt, a triethanolamine salt, and an aminomethylpropanol salt (an organic or inorganic ammonium salt); and a basic amino acid salt such as a lysine salt and an arginine salt, a choline salt, an aluminum salt, and a zinc salt. From the viewpoint of performance and availability, a sodium salt, a potassium salt, a magnesium salt, and a triethanolamine salt, and arginine are preferable, and a sodium salt is more preferable.

The content of the component A is preferably from 0.003 to 3% by mass, more preferably from 0.01 to 2% by mass, and further preferably from 0.02 to 0.5% by mass based on the mass of the starting material composition for fragrances and cosmetics.

As R3 in the general formula (B), any hydrocarbon group can be used irrespective of kinds thereof: saturated, unsaturated, linear, or branched chains. For example, R3 may be a hydrocarbon group having 9 carbon atoms, a hydrocarbon group having 11 carbon atoms, a hydrocarbon group having 13 carbon atoms, a hydrocarbon group having 15 carbon atoms, a hydrocarbon group having 17 carbon atoms, a hydrocarbon group having 19 carbon atoms, or a hydrocarbon group having 21 carbon atoms. Particularly, as an R3-C(=O)- structure, hydrocarbon groups commonly distributed in the world can be used such as a capryloyl group, an undecylenoyl group, a lauroyl group, a myristoyl group, an isomyristoyl group, a palmitoyl group, an isopalmitoyl group, a stearoyl group, an isostearoyl group, an oleyl group, a linolyl group, a linoleyl group, an arachidyl group, and a behenyl group, and mixed hydrocarbon groups of a composition derived from palm oil, palm kernel oil, or coconut oil also can be used. The groups are preferably hydrocarbon groups having 7 to 23 carbon atoms. Examples thereof include hydrocarbon groups having 1 to 29 carbon atoms. The groups are more preferably hydrocarbon groups having 7 to 17 carbon atoms. Particularly preferably, a saturated hydrocarbon groups is used, in which it is not difficult to manage prevention of air oxidation. From the economic viewpoint, the R3-C(=O)- structure is even further preferably a lauroyl group and/or a myristoyl group.

Examples of carboxylates in the general formula (B) (COOM4) include, but are not particularly limited to, a single salt or mixture of an alkali metal salt or alkaline earth metal salt such as a sodium salt, a potassium salt, a lithium salt, a magnesium salt, and a calcium salt; an amine salt such as an ammonium salt, an alkylamine salt, a monoethanolamine salt, a diethanolamine salt, a triethanolamine salt, and an aminomethylpropanol salt (an organic or inorganic ammonium salt); and a basic amino acid salt such as a lysine salt and an arginine salt, a choline salt, an aluminum salt, and a zinc salt. From the viewpoint of performance and availability, a sodium salt, a potassium salt, a magnesium salt, and a triethanolamine salt, and arginine are preferable, and a sodium salt is more preferable.

From the viewpoints of imparting high initial emulsification stability and dispersion stability to formulation for fragrances and cosmetics and economic efficiency, the proportion of the component B to the component A is preferably 0.004% or more and 7.0% or less, more preferably 0.01% or more and 3.5% or less, and further preferably 0.02% or more and 2.0% or less. When the content of the component B is 0.004% or more, emulsifiability and emulsification stability are more enhanced in the formulation system, and when the content of the component B is 7.0% or less, dissolution stability of the formulation system is further enhanced. The proportion can be measured with a HPLC analysis method as described in the following examples.

The content of the component B is preferably from 0.00003 to 0.03% by mass, more preferably from 0.0001 to 0.02% by mass, and further preferably from 0.0002 to 0.005% by mass based on the mass of the starting material composition for fragrances and cosmetics.

As R4 in the general formula (C), any hydrocarbon group can be used irrespective of kinds thereof: saturated, unsaturated, linear, or branched chains. For example, R4 may be a hydrocarbon group having 9 carbon atoms, a hydrocarbon group having 11 carbon atoms, a hydrocarbon group having 13 carbon atoms, a hydrocarbon group having 15 carbon atoms, a hydrocarbon group having 17 carbon atoms, a hydrocarbon group having 19 carbon atoms, or a hydrocarbon group having 21 carbon atoms. Particularly, as an R4-C(=O)- structure, hydrocarbon groups commonly distributed in the world can be used such as a capryloyl group, an undecylenoyl group, a lauroyl group, a myristoyl group, an isomyristoyl group, a palmitoyl group, an isopalmitoyl group, a stearoyl group, an isostearoyl group, an oleyl group, a linolyl group, a linoleyl group, an arachidyl group, and a behenyl group, and mixed hydrocarbon groups of a composition derived from palm oil, palm kernel oil, or coconut oil also can be used. Examples thereof include hydrocarbon groups having 1 to 29 carbon atoms. The groups are preferably hydrocarbon groups having 7 to 23 carbon atoms. The groups are more preferably hydrocarbon groups having 7 to 17 carbon atoms. Particularly preferably, a saturated hydrocarbon groups is used, in which it is not difficult to manage prevention of air oxidation. From the economic viewpoint, the R4-C(=O)- structure is even further preferably a lauroyl group and/or a myristoyl group.

Examples of carboxylates in the general formula (C) (COOM5, COOM6) include, but are not particularly limited to, a single salt or mixture of an alkali metal salt or alkaline earth metal salt such as a sodium salt, a potassium salt, a lithium salt, a magnesium salt, and a calcium salt; an amine salt such as an ammonium salt, an alkylamine salt, a monoethanolamine salt, a diethanolamine salt, a triethanolamine salt, and an aminomethylpropanol salt (an organic or inorganic ammonium salt); and a basic amino acid salt such as a lysine salt and an arginine salt, a choline salt, an aluminum salt, and a zinc salt. From the viewpoint of performance and availability, a sodium salt, a potassium salt, a magnesium salt, and a triethanolamine salt, and arginine are preferable, and a sodium salt is more preferable.

The content of the component C (%) is preferably 0.8% by mass or more, more preferably from 0.8% by mass or more and 8.0% by mass or less, further preferably from 1.1% by mass or more and 8.0% by mass or less, and particularly preferably from 1.5% by mass or more and 6.0% by mass or less based on the total mass of the component A and the component C. The proportion can be measured by a method as described in the following examples.

As R5 in the general formula (D), any hydrocarbon group can be used irrespective of kinds thereof: saturated, unsaturated, linear, or branched chains. For example, R5 may be a hydrocarbon group having 9 carbon atoms, a hydrocarbon group having 11 carbon atoms, a hydrocarbon group having 13 carbon atoms, a hydrocarbon group having 15 carbon atoms, a hydrocarbon group having 17 carbon atoms, a hydrocarbon group having 19 carbon atoms, or a hydrocarbon group having 21 carbon atoms. Particularly, as an R5-C(=O)- structure, hydrocarbon groups commonly distributed in the world can be used such as a capryloyl group, an undecylenoyl group, a lauroyl group, a myristoyl group, an isomyristoyl group, a palmitoyl group, an isopalmitoyl group, a stearoyl group, an isostearoyl group, an oleyl group, a linolyl group, a linoleyl group, an arachidyl group, and a behenyl group, and mixed hydrocarbon groups of a composition derived from palm oil, palm kernel oil, or coconut oil also can be used. Examples thereof include hydrocarbon groups having 1 to 29 carbon atoms. The groups are preferably hydrocarbon groups having 7 to 23 carbon atoms. The groups are more preferably hydrocarbon groups having 7 to 17 carbon atoms. Particularly preferably, a saturated hydrocarbon group is used, in which it is not difficult to manage prevention of air oxidation. From the economic viewpoint, the R5-C(=O)- structure is even further preferably a lauroyl group and/or a myristoyl group.

Examples of carboxylates in the general formula (D) (COOM7) include, but are not particularly limited to, a single salt or mixture of an alkali metal salt or alkaline earth metal salt such as a sodium salt, a potassium salt, a lithium salt, a magnesium salt, and a calcium salt; an amine salt such as an ammonium salt, an alkylamine salt, a monoethanolamine salt, a diethanolamine salt, a triethanolamine salt, and an aminomethylpropanol salt (an organic or inorganic ammonium salt); and a basic amino acid salt such as a lysine salt and an arginine salt, a choline salt, an aluminum salt, and a zinc salt. From the viewpoint of performance and availability, a sodium salt, a potassium salt, a magnesium salt, and a triethanolamine salt, and arginine are preferable, and a sodium salt is more preferable.

The content of the component D (%) is preferably 0.06% by mass or more, more preferably from 0.06% by mass or more and 3.0% by mass or less, further preferably from 0.11% by mass or more and 3.0% by mass or less, and particularly preferably from 0.14% by mass or more and 0.55% by mass or less based on the total mass of the component A and the component D. The proportion can be measured by a method as described in the following examples.

As R6 in the general formula (E), any hydrocarbon group can be used irrespective of kinds thereof: saturated, unsaturated, linear, or branched chains. For example, R6 may be a hydrocarbon group having 9 carbon atoms, a hydrocarbon group having 11 carbon atoms, a hydrocarbon group having 13 carbon atoms, a hydrocarbon group having 15 carbon atoms, a hydrocarbon group having 17 carbon atoms, a hydrocarbon group having 19 carbon atoms, or a hydrocarbon group having 21 carbon atoms. Particularly, as an R6-C(=O)- structure, hydrocarbon groups commonly distributed in the world can be used such as a capryloyl group, an undecylenoyl group, a lauroyl group, a myristoyl group, an isomyristoyl group, a palmitoyl group, an isopalmitoyl group, a stearoyl group, an isostearoyl group, an oleyl group, a linolyl group, a linoleyl group, an arachidyl group, and a behenyl group, and mixed hydrocarbon groups of a composition derived from palm oil, palm kernel oil, or coconut oil also can be used. Examples thereof include hydrocarbon groups having 1 to 29 carbon atoms. The groups are preferably hydrocarbon group having 7 to 23 carbon atoms. The groups are more preferably hydrocarbon groups having 7 to 17 carbon atoms. Particularly preferably, a saturated hydrocarbon group is used, in which it is not difficult to manage prevention of air oxidation. From the economic viewpoint, the R6-C(=O)- structure is even further preferably a lauroyl group and/or a myristoyl group.

From the viewpoints of imparting high initial emulsification stability and dispersion stability to formulation for fragrances and cosmetics and productivity, the proportion of the component E to the component A (component A/component E) is preferably from 0.005% by mass or more and 5.00% by mass or less, more preferably from 0.02% by mass or more and 3.00% by mass or less, and further preferably from 0.08% by mass or more and 0.6% by mass or less. The proportion can be measured by a method as described in the following examples.

The pH of the starting material composition for fragrances and cosmetics of the present embodiment and cosmetic agents comprising the same is, but not particularly limited to, preferably from 4.0 to 9.0, more preferably from 4.5 to 8.5, and further preferably from 5.0 to 8.0.

M1 to M7 in the formulas (A) to (D) are each independently preferably selected from the group consisting of a hydrogen atom, sodium, lithium, potassium, magnesium, ammonium, triethanolamine, and arginine.

More preferably, M1 to M7 are each independently more preferably selected from the group consisting of a hydrogen atom, sodium, lithium, potassium, magnesium, ammonium, triethanolamine, and arginine, at least one of M1 to M7 is hydrogen, and at least one of M1 to M7 is selected from the group consisting of sodium, lithium, potassium, magnesium, ammonium, triethanolamine, and arginine.

Further preferably, M1 to M7 are each independently hydrogen or sodium, at least one of M1 to M7 is hydrogen, and at least one of M1 to M7 is sodium.

[Method for producing composition]

The method for producing the composition, (particularly starting material composition for fragrances and cosmetics) of the present embodiment includes the following steps.

1) [First step] reacting glutamic acid or a salt thereof with a fatty acid chloride in a mixed solvent comprising water and an organic solvent to provide a reaction liquid containing a N-acyl glutamic acid or a salt thereof, allowing the reaction liquid to have a pH of 1 to 6 with an acid, and separating the reaction liquid into an organic layer and an aqueous layer at a temperature of 25 to 80°C.
2) [Second step] concentrating the organic layer to dryness under acidity to convert a portion of the N-acyl glutamic acid or a salt thereof into a N-acyl pyrrolidonecarboxylic acid or a salt thereof (component B) .
3) [Third step] reacting the N-acyl glutamic acid or a salt thereof with an acid anhydride to provide a N-acyl glutamic acid anhydride.
4) [Fourth step] reacting the N-acyl glutamic acid anhydride with lysine or a salt thereof to provide an acyl group-containing amino acid derivative or a salt thereof (component A).

[First step]

The acylation reaction step of the present embodiment is a step of condensation-reacting glutamic acid with a fatty acid chloride in a mixed solvent of water and an organic solvent, for example, t-butanol, acetone, methyl ethyl ketone, or isopropanol under the presence of an alkaline compound, and a crude N-acyl glutamic acid is formed by the acylation reaction.

The molar ratio of the fatty acid chloride to the glutamic acid in the acylation reaction step is preferably 1.05 or less, more preferably 1.0 or less, and further preferably 0.98 or less. As the molar ratio of the fatty acid chloride to the glutamic acid is 1.05 or less, the amount of free fatty acid to be formed tends to be reduced.

As the composition of the mixed solvent in the acylation reaction step, the volume ratio of water/organic solvent is preferably in the range of 90/10 to 20/80 and more preferably in the range of 85/15 to 50/50. With the volume ratio in the range of 90/10 to 20/80, the three components: the glutamic acid, fatty acid chloride, and alkaline compound are

likely to be compatibilized, and thus the reaction rate can be enhanced.

Although the concentration of the glutamic acid fed in the acylation reaction step is not particularly limited, the viscosity of the reaction liquid increases over time during the reaction, and thus the concentration thereof fed is preferably adjusted to an extent that stirring and mixing can be performed at the time close to the completion of the reaction.

Although the reaction temperature of the acylation reaction step is not particularly limited, the temperature is preferably in the range of -10 to 70°C, more preferably in the range of -10 to 20°C, and further preferably in the range of -5 to 10°C from the viewpoint of acceleration of the main reaction and suppression of a side reaction.

Examples of the alkaline compound used in the acylation reaction step include, but are not particularly limited to, inorganic bases such as sodium hydroxide, potassium hydroxide, calcium hydroxide, and barium hydroxide.

The pH in the acylation reaction step is preferably maintained at from 9 to 13.5 and more preferably maintained in the range of 10 to 13 from the viewpoint of acceleration of the main reaction and suppression of a side reaction.

The acylation reaction liquid is allowed to have a pH of 1 to 6, for example, with an acid such as hydrochloric acid or sulfuric acid (preferably a mineral acid), the liquid is separated into two layers: an organic layer and an aqueous layer, and the organic layer is provided. In the acylation reaction liquid, the N-acyl glutamic acid formed is present in the form of an alkali salt. Addition of an acid thereto causes the carboxyl groups in the N-acyl glutamic acid partially or entirely to be free acid, thereby separating the liquid into an organic layer and an aqueous layer (acid precipitation step). In accordance with to the pH at the time of the acid precipitation, the dissociation state of the carboxyl groups varies, and the layer separation state, that is, the mass ratio of the organic layer and the aqueous layer and the removability of inorganic salts slightly vary. Thus, the acid precipitation is performed preferably at a pH of 1 to 3 and more preferably at a pH of 1 to 2.5.

The temperature in the acid precipitation step is preferably from 25 to 80°C. The temperature is more preferably from 40 to 70°C. With a temperature of 25°C or more, there is a tendency that the time taken to reach the layer separation equilibrium can be reduced and when the equilibrium is reached, the amount of the inorganic salt remaining in the organic layer can be reduced.

[Second step]

In the second step of the present embodiment of the invention, the organic layer provided in the first step is concentrated to dryness to thereby convert a portion of the N-acyl glutamic acid into a N-acyl pyrrolidonecarboxylic acid (component B). The concentration to dryness is preferably performed with warming under normal pressure or under reduced pressure. The concentration to dryness is performed until the loss on drying reaches preferably 10% by mass or less, more preferably 3% by mass or less, and further preferably 1% by mass or less. As the acyl glutamic acid is made into an anhydride in the next step, in order to reduce the moisture, a smaller loss on drying is more preferable.

The "loss on drying" herein means the proportion of the mass to be reduced by drying. Specifically, the loss on drying is the proportion of the mass reduced when 1 g of a specimen is dried at 105°C for 3 hours, which proportion is calculated by the following expression.

$$\text{Loss on drying (\%)} = [\{\text{initial specimen mass (g)} - \text{dried specimen mass (g)}\}/\text{initial specimen mass (g)}] \times 100$$

In the concentration to dryness, the organic solvent and water are preferably distilled off under normal pressure or under reduced pressure initially at a temperature higher than that around the boiling point of the organic solvent, or a temperature around the azeotropic point of the organic solvent to be used and water. When the mass ratio of the organic solvent to water reaches substantially 3% or less, the coexisting acyl glutamic acid is more likely to be foamed, and thus careful control of reduced pressure is required under heating. Even when the organic solvent is substantially distilled off, the moisture still remains. As an actual operation, pressure reduction can be continued around the boiling point of water. Although the above operation requires foaming control, that is, mild stirring adapted to the foaming situation, in comparison with a freeze drying method, in which standing-still is continued under a set temperature, an appropriate amount of an acyl PCA is by-produced at about 10% by mass or less of the acyl glutamic acid and under a high temperature of 85°C, and this acyl PCA can be made useful for emulsifiability and dispersibility in a formulation system.

In this step, for example, as the operation, a freeze drying treatment is used. Substantially no foaming occurs during the treatment, and the drying treatment is simple because there is nothing to do manually, but no acyl PCA is observed.

As the loss on drying is reduced on the organic matter mainly based on the acyl glutamic acid, the foaming attenuates. When a temperature reached of 85°C or more is maintained for 20 minutes or more at a degree of pressure

reduction reached of 200 kPa, the loss on drying will be 10% by mass or less.

In a case in which a more efficient reduction in the loss on drying is desired, the degree of pressure reduction reached is preferably 50 kPa or less and more preferably 20 kPa or less. The temperature reached depends on the melting point and the degree of pressure reduction of the acyl glutamic acid, but is desirably 70°C or more, more preferably 95°C or more, and further preferably 105°C or more.

Setting the loss on drying to 10% by mass or less enables the component B (acyl PCA) to be by-produced from the acyl glutamic acid.

[Third step]

The solid mainly based on the acyl glutamic acid, which is provided in the second step, is reacted with a carboxylic anhydride such as acetic anhydride to provide a reaction liquid. Filtering-off from the provided reaction liquid followed by drying enables crude crystals mainly based on the acyl glutamic acid anhydride to be provided.

Here, a preferable dehydrating agent is a carboxylic anhydride like acetic anhydride, and acetic anhydride is preferably used from the economic and environmental viewpoint. After the treatment of making the acyl glutamic acid into an anhydride, excess acetic anhydride and acetic acid are removed by filtering-off. In order to enhance the purity of the acyl glutamic acid anhydride, a solid mainly based on the acyl glutamic acid anhydride can be provided by washing with ether and then petroleum ether followed by drying.

[Fourth step]

The solid mainly based on the acyl glutamic acid anhydride, provided in the third step, is reacted in accordance with Japanese Patent No. 4070768 at the rate of 1 molar equivalent of lysine per 2 molar equivalents of the acyl glutamic acid anhydride, and thus an aqueous solution mainly based on the acyl group-containing amino acid derivative represented by the general formula (A) as described below can be prepared.

The fragrances and cosmetics in the present invention are a collective term for quasi-pharmaceutical products and cosmetics referred in the Pharmaceutical and Medical Device Act, specifically, examples of quasi-pharmaceutical products can include mouth refrigerants, underarm deodorants, talcum powders, hair growth stimulants, hair removers, hair dyes, permanent wave preparations, bath preparations, medicated cosmetics, and medicated dentifrice, and examples of cosmetics can include cleansing cosmetics such as toilet soaps, facial cleansing agents (in the cream/paste, liquid/gel, granule/powder form, or for aerosol use), shampoos, and rinses; hair care cosmetics such as dyes, hair treatments (in the form of cream, mist, oil, gel and others, including split hair coating agents), hair setting agents (hair oils, hair setting lotions, curler lotions, pomade, stick pomade, bintsuke (hair setting) oil, hair spray, hair mist, hair liquid, hair foam, hair gel, and water grease); basic cosmetics such as general creams, milky lotions (such as cleansing cream, cold cream, vanishing cream, and hand cream), shaving creams (such as after shaving cream and shaving cream), skin lotions (such as hand lotions and general skin lotions), eau de colognes, shaving lotions (such as after shaving lotion and shaving lotion), cosmetic oils, and face packs; makeup cosmetics such as face powders (such as cream powder, pressed powder, powder, talcum powder, face powder paste, baby powder, body powder, and liquid powder), powder, foundations (such as in the form of cream, liquid, and solid), cheek colors, eyebrow colors, eye creams, eye shadow and mascara; perfumes such as general perfumes, solid perfumes, and powder perfumes; fragrance preparations, deodorants and deodorizers of gel-, liquid-, or ceramic container-types; suntan/sunscreen cosmetics such as suntan/sunscreen creams, suntan/sunscreen lotions, and suntan/sunscreen oils; nail cosmetics such as nail creams, nail enamels, and nail enamel removers; eye liners; lip cosmetics such as lip sticks and lip creams; oral cosmetics such as dentifrices; and bath cosmetics such as bath salts, bath oils and bubble baths.

The starting material composition for fragrances and cosmetics of the present embodiment may be used in combination with various base materials, in addition to the component A and component B to component E, in accordance with applications and purposes, as long as the object of the present invention is not impaired.

Specific examples thereof that can be contained include dispersants including natural rubbers such as gum arabic and gum tragacanth, glucosides such as saponin, cellulose derivatives such as methyl cellulose, carboxy cellulose, and hydroxymethyl cellulose, natural polymers such as lignosulfonate and shellac, anionic polymers such as polyacrylates, salts of styrene-acrylic acid copolymers, salts of vinylnaphthalene-maleic acid copolymers, sodium salts of β-naphthalenesulfonic acid-formalin condensates, and phosphonates, and non-ionic polymers such as polyvinyl alcohol, polyvinylpyrrolidone, and polyethylene glycol;

anionic surfactants such as higher fatty acid salts (soap), higher fatty acid salts in which the hydrophobic group portion has 8 to 20 carbon atoms, N-acylamino acid-type anionic surfactants, which include the acyl group having 8 to 20 carbon atoms as described above and, as a component amino acid, an amino acid such as the aforementioned acidic amino acids like glutamic acid and aspartic acid, or an amino acid such as glycine, alanine, valine, leucine,

isoleucine, proline, methionine, cystine, tryptophan, tyrosine, phenylalanine, asparagine, glutamine, serine, threonine, oxyproline, β-aminopropionic acid, γ-aminobutanoic acid, anthranilic acid, m-aminobenzoic acid, and p-aminobenzoic acid; alkyl ether carboxylates, amide ether carboxylates, alkyl sulfate ester salt (AS), polyoxyethylene alkyl ether sulfate ester salt (AES), alkyl ether sulfates, sulfates of higher fatty acid esters, sulfates of higher fatty acid alkylolamides, sulfated oils, polyoxyethylenated phenyl ether sulfates, α-olefin sulfonate (AOS), alkylbenzene sulfonates, alkylnaphthalene sulfonates, alkyl sulfonate (SAS), dialkyl sulfosuccinate, α-sulfonated fatty acid salt, alkane sulfonate, sulfonates of higher fatty acid ester, α-sulfonated fatty acid salt, sulfonates of higher fatty acid amide, N-acyl-N-alkyltaurate, N-acyl-N-methyltaurate, alkyl phosphate, alkyl ether phosphate, polyoxyethylene alkyl ether phosphate, polyoxyethylene alkylphenyl ether phosphate, and naphthalenesulfonate formalin condensate;
amphoteric surfactants such as alkylbetaines, alkylamidebetaines, alkylsulfobetaines, imidazolinium betaines, acetic acid betaines, and lecithin;
nonionic surfactants including oxidized ethylene condensate-types such as polyoxyethylene alkyl ether (AE), polyoxyethylene alkylphenyl ether, polyoxyethylene polystyrylphenyl ether, polyoxyethylene polyoxypropylene glycol, polyoxyethylene polyoxypropylene alkyl ether, polyoxyalkylene fatty acid ester, polyoxyalkylene sorbitan fatty acid ester, polyoxyalkylene fatty acid alkanolamide, polyoxyalkylene alkyl glycoside, polyoxyalkylene hydrogenated castor oil, polyoxyalkylene alkylamine, and polyoxyalkylene alkylphenyl ether, polyhydric alcohol esters such as polyhydric alcohol fatty acid partial ester, polyoxyethylene polyhydric alcohol fatty acid partial ester, polyoxyethylene fatty acid ester, (poly)glycerin fatty acid ester, polyoxyethylene hydrogenated castor oil, polyoxyethylene alkylamine, triethanolamine fatty acid partial ester, alkyl polyglycoside, propylene glycol fatty acid ester, sorbitan fatty acid ester, and sucrose fatty acid ester, fatty acid alkanolamide, sugar amine acyl compounds, triethanolamine fatty acid partial ester, fatty acid alkylolamide, and alkylamine oxide;
cationic surfactants such as primary to tertiary fatty acid amine salts, alkylammonium chloride, tetraalkyl ammonium salts, trialkylbenzyl ammonium salts, alkylpyridinium salts, alkylhydroxylethyl imidazolinium salts, dialkylmorpholinium salts, alkylisoquinolium salts, benzetonium salts, and benzalkonium salts;
polymer surfactants such as sodium alginate, starch derivatives, and tragacanth gum;
natural surfactants such as phospholipids, lecithin, lanolin, cholesterol, and saponin;
fats and oils such as avocado oil, turtle oil, corn oil, almond oil, olive oil, cacao oil, sesame oil, safflower oil, soybean oil, camellia oil, persic oil, castor oil, grape seed oil, macadamia nut oil, mink oil, cottonseed oil, Japan wax, coconut oil, egg yolk oil, palm oil, palm kernel oil, triisooctanoic acid glycerin, tri-2-ethylhexanoic acid glyceryl cholesterol fatty acid ester, wheat germ oil, sasanqua oil, linseed oil, evening primrose oil, perilla oil, peanut oil, tea seed oil, Japanese nutmeg oil, rice bran oil, Chinese tung oil, Japan tung oil, jojoba oil, germ oil, glycerin trioctanoate, glycerin triisopalmitate, horse oil, hydrogenated coconut oil, beef tallow, neat's foot oil, sheep tallow, hardened beef tallow, pork tallow, beef bone oil, Japan wax kernel oil, hydrogenated oil, Japan wax, and hydrogenated castor oil;
hydrocarbons such as liquid paraffin, petrolatum, ceresin, microcrystalline wax, isoparaffin, ozokerite, squalene, pristane, and squalane;
waxes such as beeswax, whale oil, lanolin, carnauba wax, candelilla wax, cotton wax, bayberry wax, insect wax, montan wax, bran wax, lanolin, kapok wax, lanolin acetate, liquid lanolin, sugar cane wax, isopropyl lanolate, hexyl laurate, reduced lanolin, jojoba wax, hard lanolin, shellac wax, polyoxyethylene lanolin alcohol ether, polyoxyethylene lanolin alcohol acetate, polyethylene glycol lanolate, polyoxyethylene hydrogenated lanolin alcohol ether, and derivatives thereof;
fatty acids such as lauric acid, myristic acid, palmitic acid, stearic acid, isostearic acid, oleic acid, behenic acid, undecylenic acid, lanolin fatty acid, hard lanolin fatty acid, soft lanolin fatty acid, linoleic acid, linolenic acid, eicosapentaenoic acid, and 12-hydroxystearic acid;
higher alcohols such as lauryl alcohol, cetanol, cetostearyl alcohol, stearyl alcohol, oleyl alcohol, behenyl alcohol, lanolin alcohol, hydrogenated lanolin alcohol, hexyldecanol, and octyldodecanol;
sterols such as cholesterol and phytosterol;
ester oils such as isopropyl myristate, butyl stearate, cetyl octanoate, octyldodecyl myristate, isopropyl palmitate, hexyl laurate, myristyl myristate, decyl oleate, hexyldecyl dimethyloctanoate, cetyl lactate, myristyl lactate, lanolin acetate, isocetyl stearate, isocetyl isostearate, cholesteryl 12-hydroxystearate, ethylene glycol di-2-ethylhexanoate, dipentaerythritol fatty acid ester, N-alkyl glycol monoisostearate, neopentyl glycol dicaprate, diisosterile malate, glycerin di-2-heptylundecanoate, trimethylolpropane tri-2-ethylhexanoate, trimethylolpropane triisostearate, pentaerythritol tetra-2-ethylhexanoate, glycerin tri-2-ethylhexanoate, trimethylolpropane triisostearate, cetyl-2-ethyl hexanoate, 2-ethylhexyl palmitate, glycerin trimyristate, glyceride tri-2-heptylundecanoate, methyl castor oil fatty acid ester, oleic acid oil, cetostearyl alcohol, acetoglyceride, 2-heptylundecyl palmitate, diisopropyl adipate, N-lauroyl-L-glutamic acid 2-octyldodecyl ester, di-2-heptylundecyl adipate, ethyl laurate, di-2-ethylhexyl sebacate, 2-hexyldecyl myristate, 2-hexyldecyl palmitate, 2-hexyldecyl adipate, diisopropyl sebacate, 2-ethylhexyl succinate, ethyl acetate, butyl acetate, amyl acetate, and triethyl citrate;
volatile and non-volatile silicones such as dimethyl polysiloxane, polyether modified silicone, alcohol modified silicone, methylphenyl polysiloxane, epoxy-modified silicone, fluorine-modified silicone, alkyl-modified silicone, alkyloxy-modified silicone, amino-modified silicone, polymer silicone, and cyclic silicone; polyols such as glycerin, diglycerin, polyglycerin, 1,3-butanediol, propanediol, and polyethylene glycol;

humectants including alkyl glycines such as N-methylglycine, N,N-dimethylglycine, N,N,N-trimethylglycine, N-ethylglycine, and glycyl betaine, (poly)saccharides and derivatives thereof such as sorbitol, raffinose, pyrrolidone carboxylates, lactates, hyaluronates, ceramides, trehalose, xylobiose, maltose, sucrose, glucose, and plant mucopolysaccharides, glycosamino glycanes and salts thereof such as water-soluble chitin, chitosan, pectin, chondroitin sulfate and salts thereof, amino acids and salts thereof such as glycine, serine, theonine, alanine, asparatic acid, thyrosine, valine, luecine, alginine, glutamine, and prolinic acid, saccharide amino acid compounds such as aminocarbonyl reaction products, plant extracts such as aloe and horse chestnut, and nucleic acid-related substance such as urea, uric acid, ammonia, glucosamine, creatinine, DNA and RNA;

water-soluble and oil-soluble polymers such as hydroxyethyl cellulose, carboxymethyl cellulose, hydroxyethyl cellulose hydroxypropyltrimethyl ammonium chloride ether, methyl cellulose, ethyl cellulose, hydroxypropyl cellulose, methylhydroxypropyl cellulose, soluble starch, carboxymethyl starch, methyl starch, propylene glycol alginate ester, polyvinyl alcohol, polyvinyl pyrrolidone, polyvinyl methyl ether, carboxyvinyl polymers, polyacrylates, guar gum, locust bean gum, queenseed, carrageenan, galactan, gum arabic, pectin, mannan, starch, xanthum gum, dextran, succinoglucane, cardlan, hyaluronic acid, gelatin, casein, albumin, collagen, methoxyethylene maleic anhydride copolymers, amphoteric methacrylate ester copolymers, polydimethylmethylene piperidinium chloride, polyacrylate ester copolymers, polyvinyl acetate, nitrocellulose, and silicone resin;

thickening and foaming components including cationic polymers such as cationized cellulose derivatives, cationic starch, cationized guar gum derivatives, diallyl quaternary ammonium salt/acrylamide copolymers, quaternized polyvinyl pyrrolidone derivatives, quaternized vinyl pyrrolidone/vinyl imidazole polymers, polyglycol/amine condensates, quaternized collagen polypeptide, polyethyleneimine, cationic silicone polymers, adipic acid/dimethylamino hydroxypropyl diethylene triamine copolymers, polyaminopolyamide, cationic chitin derivatives, and quaternized polymers polyethylene glycol fatty acid esters, polyoxyethylene fatty acid ester methylglycoside, and tetradecene sulfonates;

oil gelling agents such as dextrin fatty acid ester, glycerin fatty acid ester, and hydroxystearic acid;

metal ion sequestering agents such as ethylenediaminetetraacetic acid and salts thereof, hydroxyethylenediaminetriacetic acid and salts thereof, phosphoric acid, ascorbic acid, succinic acid, gluconic acid, polyphosphates, metaphosphate, and hinokithiol;

preservatives and antimicrobial agents such as paraoxybenzoate esters, benzoic acid and salts thereof, phenoxyethanol, hinokithiol, salicylic acid and salts thereof, sorbic acid and salts thereof, dehydroacetic acid and salts thereof, parachlorometacresol, hexachlorophene, boric acid, resorcin, tribromosalan, ortho-phenyl phenol, thiram, photosensitizing dye 201, halocarbane, trichlorocarbanide, tocopherol acetate, zinc pyrithione, phenol, isopropylmethylphenol, 2,4,4-trichloro-2-hydroxyphenol, hexachlorophene, chlorohexidine, benzetonium chloride, benzalkonium chloride, cetylpyridinium chloride, decalinium chloride, stearyldimethyl ammonium chloride, stearyltrimethyl ammonium chloride, cetyltrimethyl ammonium chloride, methylbenzetonium chloride, lauryltrimethyl ammonium chloride, lauroyl colamino formylmethyl pyridinium chloride, trichlosan, and biozole;

pH adjusting agents such as citric acid, malic acid, adipic acid, glutamic acid, and asparaginic acid;

dandruff and itch preventing agents such as trichlorocarbanilide, salicylic acid, zinc pyrithione, isopropylmethyl phenol, and others;

ultraviolet light absorbers such as benzophenone derivatives, paraminobenzoic acid derivatives, paramethoxycinnamic acid derivatives, salicylic acid derivatives, and others;

whitening agents such as ascorbic acid and salts thereof (alkali metal salts and alkali earth metal salts such as sodium salt, potassium salt, magnesium salt, and calcium salt, as well as ammonium salt and amino acid salt), ascorbic acid derivatives (such as alkyl L-ascorbate ester, L-ascorbic acid phosphate ester and salts thereof, L-ascorbic acid-2-sulfate ester and salts thereof, and L-ascorbic acid glucoside), alkoxysalicylic acid and salts thereof (examples of the alkoxy groups include a methoxy group, an ethoxy group, a propoxy group, an isopropoxy group, a butoxy group, and an isobutoxy group), hydroquinone glycosides and derivatives thereof (such as albutin), kojic acid and derivatives thereof, ellagic acid, chamomile extract, althea extract, liquorice extract, mulberry bark extract, raspberry extract, apple flavonoid, bran extract, vitamin E and derivatives thereof, hinokithiol, placenta extract, leucinol, camomilla ET, glutathione, clove extract, tea extract, astaxanthin, bovine placenta extract, tranexamic acid and derivatives thereof, resorcin and derivatives thereof, azulene, and $\gamma$-hydroxybutanoic acid;

blood circulation enhancers such as swertia extract, cepharanthine, vitamin E and derivatives thereof, and $\gamma$-oryzanol;

local stimulants such as capsicum tincture, ginger tincture, cantharide tincture, and benzyl nicotinate ester;

nutrients such as various vitamins and amino acids;

female hormonal agents;

hair root activators;

antiinflammatories such as glycyrrhetic acid, glycyrrhizinic acid derivatives, allantoin, azulene, aminocaproic acid, and hydrocortisone;
astringents such as zinc oxide, zinc sulfate, allantoin hydroxy aluminum, aluminum chloride, zinc sulfocarbolate, tannic acid, citric acid, and lactic acid;
cooling agents such as menthol and camphor;
antihistamines such as diphenhydramine hydrochloride, chlorpheniramine maleate, and glycyrrhizinic acid derivatives;
antioxidants such as tocopherols, BHA, BHT, gallic acid, and NDGA;
sebum inhibitors such as estradiol, estrone, and ethinylestradiol;
keratin peelers and dissolvers such as sulfur, salicylic acid, and resorcin;
α-hydroxyacids such as glycolic acid, lactic acid, malic acid, tartaric acid, and citric acid;
β-Hydroxyl acids such as salicylic acid;
cosmetic colorants such as talc, kaolin, sericite, calcium carbonate, zinc oxide, aluminum oxide, magnesium oxide, zirconium oxide, magnesium carbonate, calcium carbonate, barium sulfate, chromium oxide, chromium hydroxide, tar-based colorants, mica, (synthetic) sericite, silicon carbide, boron nitride, titanium dioxide, black titanium oxide, iron blue, red iron oxide, black iron oxide, yellow iron oxide, ultramarine, titanium-coated mica, bismuth oxychloride, bengara, binding pigments, ultramarine pink, ultramarine violet, chromium hydroxide, mica titanium, chromium oxide, aluminum cobalt oxide, carbon black, silicic anhydride, magnesium silicate, bentonite, (synthetic) mica, zirconium oxide, magnesium alumino(meta)silicate, calcium aluminosilicate, polyethylene powder, nylon powder, polymethyl methacrylate, polyethylene terephthalate-polymethyl methacrylate layered powder, acrylonitrile-methacrylic acid copolymer powder, vinylidene chloride-methacrylic acid copolymer powder, wool powder, silk powder, crystalline cellulose, N-acyllysine, polymethylsilsesquioxane powder, powdered plant fruit or peel, bismuth oxychloride, mica titanium, iron oxide-coated mica, iron oxide mica titanium, organic pigment-treated mica titanium, aluminum powder, microparticulate titanium oxide, microparticulate zinc oxide, microparticulate titanium oxide-coated mica titanium, microparticulate zinc oxide-coated mica titanium, barium sulfate-coated mica titanium, carmine, β-carotene, metal soap, chlorophyll, sunset yellow FCF, Ponceau SX, Eosine YS, tetrabromofluorescein, rhodamine B, quinoline yellow SS, quinoline yellow WS, alizanine cyanine green, quinizarin green, resolvin B, resolvin BCA, parmatone red, helindon pink CN, phthalocyanine blue, β-apo-8-carotinal, capsanthin, rilopine, bixin, crocin, canthaxanthin, shisonin, lafanine, ninocyanine, carsamine, safrole yellow, rutine, quercitine, cocoa colorant, riboflavin, laccaic acid, carminic acid, kermesic acid, alizanine, shikonin, alkannin, nikino chrome, hemoglobin, curcumin, and betanin; and purified water, and additionally pyracantha extract, n-methyl-l-serine, whey, nicotinic acid amide, diisopropylamine dichloroacetate, mevalonic acid, γ-aminobutanoic acid (including y-amino-β-hydroxybutanoic acid), althea extract, aloe extract, apricot kernel extract, turmeric extract, oolong tea extract, dried seawater, hydrolyzed wheat powder, hydrolyzed silk, carrot extract, cucumber extract, gentian extract, yeast extract, rice germ extract, comfrey extract, saponaria officinalis extract, rehmannia extract, lithospermum root extract, white birch extract, peppermint extract, swertia extract, bisabolol, propolis, luffa cylindrica extract, tilia platyphyllos flower extract, hop extract, horse chestnut extract, sapindus mukurossi peel extract, melissa officinalis leaf extract, eucalyptus leaf extract, saxifraga sarmentosa extract, rosemary extract, anthemis nobilis flower extract, royal jelly extract, seaweed, rice bran, licorice, citrus unshiu peel, angelica acutiloba, crushed prunus persica, sphingolipids, guaiazulene, and vitamin C.

## Examples

Hereinafter, the present invention will be described in more detail with reference to Examples and Comparative examples, but the technical scope of the present invention is not limited thereto.

Various values in Examples may be preferable lower limit values or upper limit values in the embodiment of the present invention or may be appropriately combined with a value of the same kind (including the lower limit value and upper limit value of a numerical range) in the embodiment of the present invention described above to make a preferable numerical range. Two values of the same kind in Examples may be appropriately combined to make a preferable numerical range.

### [Production Example 1]

To a mixed solution of 104 g (0.56 mol) of monosodium L-glutamate monohydrate, 265.2 g of pure water, and 69.6 g (0.56 mol as sodium hydroxide) of a 32% sodium hydroxide aqueous solution, 93.2 g of an 87% by mass t-butanol aqueous solution was added. With this solution maintained at 3°C, 117.8 g (0.54 mol) of lauroyl chloride was added dropwise under stirring while the pH was adjusted to 12 with 32% sodium hydroxide.

Next, 75% sulfuric acid was added dropwise to adjust the pH value of the solution to 2 and the temperature of the solution to 65°C. The solution was allowed to stand for an hour and separated into an organic layer and an aqueous

layer, and then the organic layer was recovered.

The component weight ratio of the solid mainly based on lauroyl glutamic acid/t-butanol/water in the organic layer at this time was 62/24/14.

[Production Example 2]

To a mixed solution of 104 g (0.56 mol) of monosodium L-glutamate monohydrate, 145.8 g of pure water, and 69.6 g (0.56 mol as sodium hydroxide) of a 32% sodium hydroxide aqueous solution, 126 g of acetone was added. With this solution maintained at 3°C, 117.8 g (0.54 mol) of lauroyl chloride was added dropwise under stirring while the pH was adjusted to 12 with 32% sodium hydroxide.

Next, 75% sulfuric acid was added dropwise to adjust the pH value of the solution to 2 and the temperature of the solution to 50°C. The solution was allowed to stand for an hour and separated into an organic layer and an aqueous layer, and then the organic layer was recovered.

The component weight ratio of the solid mainly based on lauroyl glutamic acid/acetone/water in the organic layer at this time was 55/30/15.

[Production Example 3]

To a mixed solution of 104 g (0.56 mol) of monosodium L-glutamate monohydrate, 265.2 g of pure water, and 69.6 g (0.56 mol as sodium hydroxide) of a 32% sodium hydroxide aqueous solution, 93.2 g of an 87% by mass t-butanol aqueous solution was added. With this solution maintained at 3°C, 26.6 g (0.11 mol) of myristoyl chloride was added dropwise under stirring, and subsequently 94.2 g (0.43 mol) of lauroyl chloride was added dropwise under stirring while the pH was adjusted to 12 with 32% sodium hydroxide.

Next, 75% sulfuric acid was added dropwise to adjust the pH value of the solution to 2 and the temperature of the solution to 65°C. The solution was allowed to stand for an hour and separated into an organic layer and an aqueous layer, and then the organic layer was recovered.

The component weight ratio of the solid mainly based on acyl glutamic acid (lauroyl glutamic acid and myristoyl glutamic acid)/t-butanol/water in the organic layer at this time was 62/27/11.

<HPLC analysis method>

In Examples and Comparative Examples containing the component B described below, the component A and component B were measured from the peak area obtained by an analysis using HPLC described below.

Detector: UV detector (205 nm)
As a measurement instrument, for example, SPD-10Avp, SPD-10AVvp, SPD-20A, SPD-20AV, or the like of Shimadzu Corporation can be used.
Separation tube: a stainless tube of 6 mm in inner diameter and 150 mm in length was filled with silica gel of 5 μm in particle diameter and 12 nm in pore diameter modified with octadecylsilyl groups.
Separation tube temperature: a constant temperature around 60°C
Mobile phase: methanol/water/phosphoric acid = 2000/400/0.31
Flow rate: a constant amount around 0.8 mL per minute

More specifically, the component ratio was calculated from the peak area of each of substances detected such as the component A and component B, obtained by an analysis using HPLC. First, the obtained loss on drying was subtracted from the total component ratio 100%, and the value obtained by the subtraction was allotted in accordance with the peak area ratio obtained by the HPLC analysis. Separately, the component B proportion (%) was calculated by the following expression. This proportion corresponds to "Component B proportion (%)" in the following Tables.

$$\text{Component B proportion (\%)} = [\text{peak area of component B/peak area of component A}] \times 100$$

When UV detection is used as described above, the peak sensitivity of the acyl PCA (component B) is higher than that of other substance, and thus a high measurement sensitivity can be kept also in the component B (%) proportion.

[Example 1]

100 g of the organic layer provided in Production Example 1, which had a component weight ratio of the solid such as lauroyl glutamic acid/t-butanol/water of 62/24/14 was allowed to warm under normal pressure with gentle stirring until the liquid temperature reached 90°C. Then, the layer was allowed to warm while the pressure was gradually reduced, the degree of pressure reduction was gently raised so as to prevent excess foaming, and at a degree of pressure reduction reached of 20 kPa, the temperature reached of 105°C was maintained for 20 minutes. Then, the pressure was gradually returned to normal pressure, and the melt in the pot was recovered. The provided solid was placed in the refrigerator overnight and pulverized with a juicing mixer. As a result, a solid of 56.6 g was provided. The loss on drying of this solid was 0.25%, and the results of the HPLC analysis were 94.9% of lauroyl glutamic acid, 3.7% of lauroyl PCA, and 0.44% of lauric acid. No other marked impurities were detected.

54.0 g of the solid was dissolved in 36.4 g of acetic anhydride at 50°C, 18.2 g of acetic anhydride was further added thereto, and the reaction was performed still at 50°C for 3 hours. Then, the temperature was cooled to room temperature, and the product was filtered off from the reaction liquid to thereby provide crude crystals mainly based on N-lauroyl-L-glutamic anhydride. These crystals were washed with 80 ml of ether followed by 15 ml of petroleum ether and dried to provide 34.0 g of a solid. The loss on drying of this solid was 0.24%, and the results of the HPLC analysis were 95.2% of lauroyl glutamic anhydride, 2.3% of lauroyl PCA, and 2.2% of lauroyl glutamic acid. No lauric acid was detected. No other marked impurities were detected.

Further, 9.1 g (0.05 mol) of L-lysine chloride was mixed with 57 g of water. A reaction was performed by adding this solution to 31.1 g of the solid, which was provided by washing and drying as described above, over 2 hours under stirring while the pH range thereof was adjusted with a 25% sodium hydroxide aqueous solution to 10 to 11 and the reaction temperature was maintained at 5°C. After the stirring was continued for further 30 minutes, t-butanol was added thereto at a concentration in the solution of 20% by mass. Then, 75% sulfuric acid was added dropwise to adjust the pH of the solution to 2, and the temperature of the solution was adjusted to 65°C. After completion of the dropping, stirring was stopped, and the solution was allowed to stand at 65°C for 20 minutes. Then the solution was separated into an organic layer and an aqueous layer, and the organic layer was removed therefrom. Unreacted lysine was substantially recovered in the aqueous layer. After the provided organic layer was subjected to the same water washing operation repeatedly, the solvent was removed from the provided organic layer, and an aqueous solution having a solid content of 30% by mass and a pH of 7 (25°C) was prepared by neutralization with sodium hydroxide. The solution was distilled under reduced pressure while water was added thereto, and the distillation under reduced pressure was continued at a degree of pressure reduction of 13 to 22 kPa and a solution temperature of 45 to 60°C until the concentration of t-butanol in the solution reached 10 ppm by mass or less. Finally provided was a 32% by weight aqueous solution mainly based on a composition containing an acyl compound represented by the formula (A), the pH of which solution was 7 (25°C). The content of the component A was 96.9%, the content of the acyl PCA as the component B was 0.031%, and the content of lauroyl glutamic acid was 2.98%. No other marked impurities were detected. The proportion of the acyl PCA (%) according to the HPLC analysis method described above was 0.032%.

[Comparative Example 1]

100 g of the organic layer provided in Production Example 1, which had a component weight ratio of the solid such as lauroyl glutamic acid/t-butanol/water of 62/24/14, was freeze-dried.
For preliminary freezing, the shelf circulation temperature was set to -60°C, and after the specimen temperature reached -40°C, the organic layer was cooled for 2 hours. The shelf circulation temperature was set to -40°C, and the layer was dried for 2 hours. Then, the temperature was set to -10°C, and the layer was dried at approximately 5 Pa. Thereafter, the set temperature was raised to 0°C, 10°C, and 30°C.

After a product temperature of 27.5°C and a degree of vacuum reached of 1 Pa or less were confirmed, the vacuum was released with dry nitrogen. The drying step above was performed over a time required of 40 hours.

The provided solid was in the form of a plate, which was pulverized with a juicing mixer to thereby provide 57.1 g of a solid. The loss on drying of this solid was 1.2%, and the results of the HPLC analysis were 98.1% of lauroyl glutamic acid, no detection of lauroyl PCA, and 0.40% of lauric acid. No other marked impurities were detected.

54.0 g of the solid was dissolved in 36.4 g of acetic anhydride at 50°C, 18.2 g of acetic anhydride was further added thereto, and the reaction was performed still at 50°C for 3 hours. Then, the temperature was cooled to room temperature, and the product was filtered off from the reaction liquid to thereby provide crude crystals of N-lauroyl-L-glutamic anhydride. These crystals were washed with 80 ml of ether followed by 15 ml of petroleum ether and dried to provide 34.3 g of a solid. The loss on drying of this solid was 1.2%, and the results of the HPLC analysis were 96.6% of lauroyl glutamic anhydride and 2.1% of lauroyl glutamic acid, and neither lauroyl PCA nor lauric acid was detected. No other marked impurities were detected.

Further, 9.1 g (0.05 mol) of L-lysine chloride was mixed with 57 g of water. Thereafter, preparation of the

component A was continued by the same procedure as in Example 1, and as a result, a 32% by weight aqueous solution mainly based on a composition containing the component A was provided, the pH of which solution was 7 (25°C). At this time, the content of the component A was 97.1%, and the content of lauroyl glutamic acid was 2.62%. Neither lauroyl PCA nor lauric acid was detected, and no other marked impurities were detected. The proportion of the acyl PCA (%) according to the HPLC analysis method was 0%.

[Example 2]

115.0 g of the organic layer provided in Production Example 2, which had a component weight ratio of the solid such as lauroyl glutamic acid/ acetone/water of 55/30/15, was allowed to warm under normal pressure with gentle stirring until the liquid temperature reached 50°C. Then, the layer was allowed to warm while the pressure was gradually reduced, the degree of pressure reduction was gently raised so as to prevent excess foaming, and at a degree of pressure reduction reached of 20 kPa, the temperature reached of 105°C was achieved. At a degree of pressure reduction reached of 20 kPa, the temperature reached of 105°C was maintained for 80 minutes. Then, the pressure was gradually returned to normal pressure, and the melt in the pot was recovered. The time required from the start to completion of the concentration was 10 hours. The provided solid was placed in the refrigerator overnight and pulverized with a juicing mixer. As a result, a solid of 58.9 g was provided. The loss on drying of this solid was 0.21%, and the results of the HPLC analysis were 83.5% of lauroyl glutamic acid, 15.6% of lauroyl PCA, and 0.14% of lauric acid. No other marked impurities were detected.

54.0 g of the solid was dissolved in 36.4 g of acetic anhydride at 50°C, 18.2 g of acetic anhydride was further added thereto, and the reaction was performed still at 50°C for 3 hours. Then, the temperature was cooled to room temperature, and the product was filtered off from the reaction liquid to thereby provide crude crystals of N-lauroyl-L-glutamic anhydride. These crystals were washed with 80 ml of ether followed by 15 ml of petroleum ether and dried to provide 34.2 g of a solid. The loss on drying of this solid was 0.21%, the results of the HPLC analysis were 83.7% of lauroyl glutamic anhydride, 1.9% of lauroyl glutamic acid, and 13.4% of lauroyl PCA, and no lauric acid was detected. No other marked impurities were detected.

Further, 9.1 g (0.05 mol) of L-lysine chloride was mixed with 57 g of water. Thereafter, preparation of the component A was continued by the same procedure as in Example 1, and as a result, a 32% by weight aqueous solution mainly based on a composition containing the component A was provided, the pH of which solution was 7 (25°C). At this time, the content of the component A was 95.3%, the content of the acyl PCA as the component B was 1.27%, and the content of lauroyl glutamic acid was 3.33%. No other marked impurities were detected. The proportion of the acyl PCA (%) according to the HPLC analysis method described above was 1.33%.

[Comparative Example 2]

115.0 g of the organic layer provided in Production Example 2, which had a component weight ratio of the solid such as lauroyl glutamic acid/acetone/water of 55/30/15, was freeze-dried.

For preliminary freezing, the shelf circulation temperature was set to -60°C, and after the specimen temperature reached -40°C, the organic layer was cooled for 2 hours. The shelf circulation temperature was set to -40°C, and the layer was dried for 2 hours. Then, the temperature was set to -10°C, and the layer was dried at approximately 12 Pa. Thereafter, the set temperature was raised to 0°C, 15°C, and 30°C.

After a product temperature of 27.5°C and a degree of vacuum reached of 1 Pa or less were confirmed, the vacuum was released with dry nitrogen. The drying step above was performed over a time required of 70 hours.

The provided solid was in the form of a plate, which was pulverized with a juicing mixer to thereby provide 59.5 g of a solid. The loss on drying of this solid was 0.51%, the results of the HPLC analysis were 98.9% of lauroyl glutamic acid, and neither lauroyl PCA nor lauric acid was detected. No other marked impurities were detected.

54.0 g of the solid was dissolved in 36.4 g of acetic anhydride at 50°C, 18.2 g of acetic anhydride was further added thereto, and the reaction was performed still at 50°C for 3 hours. Then, the temperature was cooled to room temperature, and the product was filtered off from the reaction liquid to thereby provide crude crystals of N-lauroyl-L-glutamic anhydride. These crystals were washed with 80 ml of ether followed by 15 ml of petroleum ether and dried to provide 34.2 g of a solid. The loss on drying of this solid was 1.2%, the results of the HPLC analysis were 96.0% of lauroyl glutamic anhydride and 2.4% of lauroyl glutamic acid, and neither lauroyl PCA nor lauric acid was detected. No other marked impurities were detected.

Further, 9.1 g of L-lysine chloride (0.05 mol) was mixed with 57 g of water. Thereafter, preparation of the component A was continued by the same procedure as in Example 1, and as a result, a 32% by weight aqueous solution mainly based on a composition containing the component A was provided, the pH of which solution was 7 (25°C). At this time, the content of the component A was 96.3%, and the content of lauroyl glutamic acid was 3.30%. Neither lauroyl PCA nor lauric acid was detected, and no other marked impurities were detected. The proportion of the acyl PCA (%)

according to the HPLC analysis method was 0%.

[Example 3]

100.0 g of the organic layer provided in Production Example 3, which had a component weight ratio of the solid such as acyl glutamic acid/t-butanol/water of 62/27/11, was allowed to warm under normal pressure with gentle stirring until the liquid temperature reached 70°C. Then, the layer was allowed to warm while the pressure was gradually reduced, the degree of pressure reduction was gently raised so as to prevent excess foaming, and at a degree of pressure reduction reached of 20 kPa, the temperature reached of 115°C was achieved and maintained for 20 minutes. Then, the pressure was gradually returned to normal pressure, and the melt in the pot was recovered. The provided solid was placed in the refrigerator overnight and pulverized with a juicing mixer. As a result, a solid of 55.5 g was provided. The loss on drying of this solid was 0.14%, and the results of the HPLC analysis were 90.9% of acyl glutamic acid, 7.7% of acyl PCA, and 0.74% of free fatty acid (total of lauric acid and myristic acid). No other marked impurities were detected.

54.0 g of the solid was dissolved in 36.4 g of acetic anhydride at 50°C, 18.2 g of acetic anhydride was further added thereto, and the reaction was performed still at 50°C for 3 hours. Then, the temperature was cooled to room temperature, and the product was filtered off from the reaction liquid to thereby provide crude crystals of N-acyl-L-glutamic anhydride. These crystals were washed with 80 ml of ether followed by 15 ml of petroleum ether and dried to provide 33.7 g of a solid. The loss on drying of this solid was 0.20%, the results of the HPLC analysis were 91.6% of acyl glutamic acid anhydride, 1.7% of acyl glutamic acid, and 6.1% of acyl PCA, and no free fatty acid was detected. No other marked impurities were detected.

Further, 9.1 g of L-lysine chloride (0.05 mol) was mixed with 57 g of water. Thereafter, preparation of the component A was continued by the same procedure as in Example 1, and as a result, a 32% by weight aqueous solution mainly based on a composition containing the component A was provided, the pH of which solution was 7 (25°C). At this time, the content of the component A was 96.2%, the content of the acyl PCA as the component B was 0.293%, and the content of the acyl glutamic acid was 2.61%. No free fatty acid was detected. No other marked impurities were detected. The proportion of the acyl PCA (%) according to the HPLC analysis method described above was 0.312%.

[Comparative Example 3]

100 g of the organic layer provided in Production Example 3, which had a component weight ratio of the solid such as acyl glutamic acid/t-butanol/water of 62/27/11, was freeze-dried.

For preliminary freezing, the shelf circulation temperature was set to -60°C, and after the specimen temperature reached -40°C, the organic layer was cooled for 2 hours. The shelf circulation temperature was set to -40°C, and the layer was dried for 2 hours. Then, the temperature was set to -10°C, and the layer was dried at approximately 5 Pa. Thereafter, the set temperature was raised to 0°C, 20°C, and 30°C.

After a product temperature of 27.5°C and a degree of vacuum reached of 1 Pa or less were confirmed, the vacuum was released with dry nitrogen. The drying step above was performed over a time required of 55 hours.

The provided solid was in the form of a plate, which was pulverized with a juicing mixer to thereby provide 57.2 g of a solid. The loss on drying of this solid was 0.61%, and the results of the HPLC analysis were 98.6% of acyl glutamic acid, no detection of acyl PCA, and 0.54% of free fatty acid. No other marked impurities were detected.

54.0 g of the solid was dissolved in 36.4 g of acetic anhydride at 50°C, 18.2 g of acetic anhydride was further added thereto, and the reaction was performed still at 50°C for 3 hours. Then, the temperature was cooled to room temperature, and the product was filtered off from the reaction liquid to thereby provide crude crystals of N-acyl-L-glutamic anhydride. These crystals were washed with 80 ml of ether followed by 15 ml of petroleum ether and dried to provide 32.8 g of a solid. The loss on drying of this solid was 1.5%, the results of the HPLC analysis were 96.0% of acyl glutamic acid anhydride and 2.1% of acyl glutamic acid, and acyl PCA and free fatty acid were not detected. No other marked impurities were detected.

Further, 9.1 g (0.05 mol) of L-lysine chloride was mixed with 57 g of water. Thereafter, preparation of the component A was continued by the same procedure as in Example 1, and as a result, a 32% by weight aqueous solution mainly based on a composition containing the component A was provided, the pH of which solution was 7 (25°C). At this time, the content of the component A was 96.6%, and the content of acyl glutamic acid was 3.15%. Neither acyl PCA nor free fatty acid was detected, and no other marked impurities were detected. The proportion of the acyl PCA (%) according to the HPLC analysis method was 0%.

[Example 4]

An organic layer having a component weight ratio of the solid such as lauroyl glutamic acid/t-butanol/water of 64/25/11 was provided by separately performing again an operation equivalent to that in Production Example 1. 125 g

of this organic layer was allowed to warm under normal pressure with gentle stirring until the liquid temperature reached 90°C. Then, the layer was allowed to warm while the pressure was gradually reduced, the degree of pressure reduction was gently raised so as to prevent excess foaming, and at a degree of pressure reduction reached of 40 kPa, the temperature reached of 105°C was maintained for 120 minutes. Then, the pressure was gradually returned to normal pressure, and the melt in the pot was recovered. The provided solid was placed in the refrigerator overnight and pulverized with a juicing mixer. As a result, a solid of 70.6 g was provided. The loss on drying of this solid was 0.20%, and the results of the HPLC analysis were 80.7% of lauroyl glutamic acid, 14.1% of lauroyl PCA, and 4.7% of lauric acid. No other marked impurities were detected.

54.0 g of the solid was dissolved in 36.4 g of acetic anhydride at 50°C, 18.2 g of acetic anhydride was further added thereto, and the reaction was performed still at 50°C for 3 hours. Then, the temperature was cooled to room temperature, and the product was filtered off from the reaction liquid to thereby provide crude crystals mainly based on N-lauroyl-L-glutamic anhydride. These crystals were washed with 80 ml of ether followed by further 80 ml of ether again and dried to provide 32.7 g of a solid. The loss on drying of this solid was 0.19%, the results of the HPLC analysis were 84.5% of lauroylglutamic anhydride, 13.0% of lauroyl PCA, and 1.9% of lauroyl glutamic acid, and no lauric acid was detected. No other marked impurities were detected.

Further, 9.1 g (0.05 mol) of L-lysine chloride was mixed with 57 g of water. A reaction was performed by adding this solution to 32.7 g of the solid, which was provided by washing and drying as described above, over 2 hours under stirring while the pH range thereof was adjusted with a 25% sodium hydroxide aqueous solution to 10 to 11 and the reaction temperature was maintained at 5°C. After the stirring was continued for further 30 minutes, t-butanol was added thereto at a concentration in the solution of 20% by mass. Then, 75% sulfuric acid was added dropwise to adjust the pH of the solution to 2, and the temperature of the solution was adjusted to 65°C. After completion of the dropping, stirring was stopped, and the solution was allowed to stand at 65°C for 20 minutes. Then the solution was separated into an organic layer and an aqueous layer, and the organic layer was removed therefrom. Unreacted lysine was substantially recovered in the aqueous layer. After the provided organic layer was subjected to the same water washing operation repeatedly, the solvent was removed from the provided organic layer, and an aqueous solution having a solid content of 30% by mass and a pH of 7 (25°C) was prepared by neutralization with sodium hydroxide. The solution was distilled under reduced pressure while water was added thereto, and the distillation under reduced pressure was continued at a degree of pressure reduction of 13 to 22 kPa and a solution temperature of 45 to 60°C until the concentration of t-butanol in the solution reached 10 ppm by mass or less. Finally provided was a 32% by weight aqueous solution mainly based on a composition containing an acyl compound represented by the formula (A), the pH of which solution was 7 (25°C). The content of the component A was 91.2%, the content of the acyl PCA as the component B was 6.25%, and the content of lauroyl glutamic acid was 2.32%. No other marked impurities were detected. The proportion of the acyl PCA (%) according to the HPLC analysis method described above was 6.85%.

<HPLC analysis method and solid content measurement method>

For calculating the proportion of the component C and the component D to the component A, the purity was calculated from the peak area obtained by the following analysis using HPLC.

Detector: RI detector
Separation tube: a stainless tube of 6 mm in inner diameter and 150 mm in length was filled with silica gel of 5 μm in particle diameter and 12 nm in pore diameter modified with octadecylsilyl groups.
Separation tube temperature: a constant temperature around 60°C
Mobile phase: methanol/water/phosphoric acid = 2000/400/0.31
Flow rate: 0.8 mL per minute

Subsequently, component C/component A + C and component D/component A + D are represented by the following expressions, respectively corresponding to "Component C mass (%)" and "Component D mass (%)" in the following tables.

Component C/component A + C = [pure content of component C (g)/pure content of component A (g) + pure content of component C (g)] × 100 (%)

$$\text{Component D/component A + D} = [\text{pure content of component D (g)/pure content of component A (g) + pure content of component D (g)}] \times 100\ (\%)$$

<Preparation of high-purity component A>

A freeze-dried product of Pellicer L-30 manufactured by Asahi Kasei Finechem Co., Ltd. (moisture: 2.0%) or a freeze-dried product thereof produced by changing the acid chloride in Example 1 was used as a starting material to perform the following operation. The above freeze-dried product, distilled water, and t-butanol were stirred in a weight ratio of 45/33/22 at 60°C. After 60°C was reached, sulfuric acid was gently added thereto, addition of sulfuric acid was continued at 65°C until a pH of 2 was reached. After 30 minutes, the stirring was stopped, and the solution was allowed to stand. After 30 minutes of standing-still, the lower layer separated (aqueous layer) was removed. The lower layer (aqueous layer) was weighed, a 20% by weight t-butanol aqueous solution (weight ratio of t-butanol/distilled water: 2/8) of the same weight was added to the upper layer and subjected to stirring at 60°C. After 30 minutes, the stirring was stopped, and the solution was allowed to stand for 30 minutes. The lower layer was removed again. After a 20% by weight t-butanol aqueous solution of the same weight as that of the lower layer described above was injected, the step of stirring, standing-still, and removal of the lower layer (water washing and demineralization step) was repeated further twice, that is, 3 times in total. The organic layer subjected to the water washing and demineralization step was freeze-dried.

The above dried product was washed with 100 ml of ether followed by 50 ml of petroleum ether and dried. This was performed again to thereby provide the component A. This was analyzed with the HPLC described above, but no other components having a peak proportion of 0.1% or more were detected.

<Preparation of high-purity component B>

Under conditions of nitrogen atmosphere and 10°C, 90 mmol of pyroglutamic acid, 180 mmol of triethylamine, and 150 mL of acetonitrile were stirred and mixed in a flask. Next, a solution obtained by adding 90 mmol of various acid chlorides to 60 mL of acetonitrile was added thereto, the flask was removed from the ice bath, and the reaction mixture was stirred at room temperature for 2 hours. After filtered through a glass filter, the reaction mixture was concentrated under reduced pressure using an evaporator. After addition of 150 mL of water and 120 mL of 1 M hydrochloric acid thereto, the residue was extracted with 100 ml of ethyl acetate 4 times. The organic layer combined was dried over magnesium sulfate and then concentrated in vacuo. The residue was recrystallized with heptane/ethyl acetate = 5:4 (90 mL) to provide acyl pyroglutamic acid at a purity of 95% or more.

<Preparation of high-purity component C>

Aminosurfact ALMS-P1 manufactured by Asahi Kasei Finechem Co., Ltd. or a composition produced by changing the acid chloride in the same manner as in Example 1 was subjected repeatedly to the water washing operation twice. Then, a composition was prepared by removing the solvent from the provided organic layer, neutralizing the solvent with sodium hydroxide so as to give an aqueous solution having a solid content of 30% by mass and a pH of 7 (25°C), and drying the solution. The composition as the starting material was subjected to the following operation. The above product, distilled water, and t-butanol were stirred in a weight ratio of 45/33/22 at 60°C. After 60°C was reached, sulfuric acid was gently added thereto, addition of sulfuric acid was continued at 65°C until a pH of 2 was reached. After 30 minutes, the stirring was stopped, and the solution was allowed to stand. After 30 minutes of standing-still, the lower layer separated (aqueous layer) was removed. The lower layer (aqueous layer) was weighed, a 20% by weight t-butanol aqueous solution (weight ratio of t-butanol/distilled water: 2/8) of the same weight was added to the upper layer and subjected to stirring at 60°C. After 30 minutes, the stirring was stopped, and the solution was allowed to stand for 30 minutes. The lower layer was removed again. After the 20% by weight t-butanol aqueous solution of the same weight as that of the lower layer described above was injected, the step of stirring, standing-still, and removal of the lower layer (water washing and demineralization step) was repeated further twice, that is, 3 times in total. The organic layer subjected to the water washing and demineralization step was freeze-dried.

The above dried product was washed with 120 ml of ether followed by 60 ml of petroleum ether and dried. This was performed again to thereby provide the component C. This was analyzed with the HPLC described above, but no other components having a peak proportion of 0.1% or more were detected.

<Obtainment of high-purity component D>

Caprylic acid (O0027), lauric acid (L0011), myristic acid (M0476), palmitic acid (P1145), stearic acid (S0163), and behenic acid (B1248) were obtained from Tokyo Chemical Industry Co., Ltd.

<Obtainment of high-purity component E>

Caprylic acid amide (O237203), lauric acid amide (516472), myristic acid amide (B22610), palmitic acid amide (QE-1627), stearic acid amide (192-04015), and behenic acid amide (B131150) were obtained from FUJIFILM Wako Pure Chemical Corporation.

<LC-MS analysis method>

In Examples and Comparative Examples, the purity of the component E was confirmed with an analysis using the following LC-M.

(LC-MS apparatus)

Separation tube: a stainless tube of 2 mm in inner diameter and 30 mm in length was filled with phenyl group-modified particles having a particle diameter of 3 μm.

Separation tube temperature: a constant temperature around 40°C
Mobile phase: gradient elution method of 10 mM ammonium acetate aqueous solution and acetonitrile
Flow rate: a constant amount around 0.3 mL per minute
As a measurement instrument, for example, LCMS-9030 or the like of Shimadzu Corporation can be used.
Ionization: ESI+
Scan range: m/z 50 to 1200
M+H: 200.203

<HPLC analysis method>

In Examples and Comparative Examples, when the proportion of the component E and the component A was calculated, the purity was confirmed by an analysis using the following HPLC.

Detector: UV detector (205 nm)
As a measurement instrument, for example, SPD-10Avp, SPD-10AVvp, SPD-20A, SPD-20AV, or the like of Shimadzu Corporation can be used.
Separation tube: a stainless tube of 6 mm in inner diameter and 150 mm in length was filled with silica gel of 5 μm in particle diameter and 12 nm in pore diameter modified with octadecylsilyl groups.
Separation tube temperature: a constant temperature around 60°C
Mobile phase: methanol/water/phosphoric acid = 2000/400/0.31
Flow rate: a constant amount around 0.8 mL per minute

More specifically, component E/component A is represented by the following expression, corresponding to "Component E mass (%)" in the following Tables.

$$\text{Component E/component A} = [\text{pure content of component E (g)/pure content of component A (g)}] \times 100(\%)$$

Cocamide MEA used in Examples and Comparative Examples was AMISOL CME from Kawaken Fine Chemicals Co., Ltd., and Lauramide MEA used therein was AMISOL LME from Kawaken Fine Chemicals Co., Ltd.

The 32% aqueous solution of Example 1 in Examples 27, 30, 33, 36, 42, 45, 48, 51, 54, 57, 60, 63, 66, 69, 72, 75, 78, 81, 84, and 87, the 32% aqueous solution in Example 2 in Examples 28, 31, 34, 37, 43, 46, 49, 52, 55, 58, 61, 64, 67, 70, 73, 76, 79, 82, 85, and 88, or the 32% aqueous solution of Example 4 in Examples 29, 32, 35, 38, 44, 47, 50, 53, 56, 59, 62, 65, 68, 71, 74, 77, 80, 83, 86, and 89 was mixed with the above high-purity component A, component C, component D, and component E for preparation so as to achieve the counter salt pH listed in the tables. In other

Examples and Comparative Examples, the high-purity component A and component C to component E were accurately weighed, an amount calculated of sodium hydroxide or potassium hydroxide or triethanolamine or arginine, which may become a counter salt, was added to the provided solid powder to prepare aqueous solutions having a pH and a solid content adjusted. These aqueous solutions were adjusted to have a pH finally specified and a solid content of 32%. In Comparative Examples 7 to 9, 10, 12, 14, 16, 18, 20, 22, 24, 26, 28, and 30, the component A was replaced by Polysorbate 60 of the same amount, and the proportions of the component B to component E were described as the proportions in the case where Polysorbate 60 was the component A. 1% of Na stearoyl glutamate in Example 9 and 3% of myristic acid and 2% of palmitic acid in Example 10 were added in place of purified water.

<Emulsification stability confirmation test 1>

The following composition was prepared.

| Composition | wt% |
|---|---|
| • 32% aqueous solution of Examples or Comparative Examples | 0.09 |
| • Oil (ethylhexyl palmitate, olive oil, mineral oil, cyclomethicone, dimethicone) | 20.0 |
| • Carbomer (Carbopol 981) | 0.2 |
| • 30 wt/v% NaOH aqueous solution | 0.28 |
| • Purified water | 79.43 |

The composition is prepared in accordance with the following order of 1 to 4.

1. Carbomer is dissolved in purified water.
2. The Carbomer aqueous solution is adjusted with a KOH or triethanolamine or arginine or NaOH aqueous solution to have a specified pH.
3. The 32% aqueous solution of Examples or Comparative Examples and the oils each are added thereto.
4. The resultant is emulsified at room temperature for 5 minutes using a homomixer (6000 rpm).

The above composition was stored at 55°C and visually confirmed after 2 months, after 2.5 months, and after 3.0 months.

Evaluation criteria

5: Emulsified over the entire liquid depth
4: Emulsified over 90% or more of the liquid depth
3: Emulsified over 80% or more of the liquid depth
2: Emulsified over 70% or more of the liquid depth
1: Emulsified liquid depth is less than 70%

<Emulsification stability confirmation test 2>

The following composition was prepared.

| Composition | wt% |
|---|---|
| • 32% aqueous solution of Examples or Comparative Examples | 0.09 |
| • Mineral oil (5, 8 to 8.9 mm2/s) | 16.0 |
| • Solid oil (cetostearyl alcohol, myristyl alcohol) | 4.0 |
| • Carbomer (Carbopol 981) | 0.2 |
| • 30 wt/v% NaOH aqueous solution | 0.28 |
| • Purified water | 79.43 |

The composition is prepared in accordance with the following order of 1 to 4.

1. Carbomer is dissolved in purified water, and the Carbomer aqueous solution is adjusted with a KOH or triethanolamine or arginine or NaOH aqueous solution to have a specified pH.
2. The 32% aqueous solution of Examples or Comparative Examples and purified water was added to thereto to give an aqueous phase. This aqueous phase is warmed to 70°C.
3. Mineral oil and solid oil as an oil phase are warmed to 70°C.
4. The aqueous phase and the oil phase are mixed, and the mixture is stirred and warmed with a homomixer at 3000 rpm to 80°C.
5. Emulsification is performed with a homomixer at 6000 rpm for 5 minutes.
6. Stirring and cooling are performed with a homomixer at 3000 rpm to 25°C.
7. Deaeration is performed.

The above composition was stored at 55°C, allowed to stand for 2 months, 2.5 months, and 3.0 months, and its emulsifiability was visually confirmed.

Evaluation criteria

5: Emulsified over the entire liquid depth
4: Emulsified over 90% or more of the liquid depth
3: Emulsified over 80% or more of the liquid depth
2: Emulsified over 70% or more of the liquid depth
1: Emulsified liquid depth is less than 70%

<Dispersibility stability confirmation test>

Titanium oxide was added at a concentration of 1% to an aqueous solution having a solid content of 0.0075% each of Examples and Comparative Examples, and a KOH or triethanolamine or arginine or NaOH aqueous solution was added thereto so as to achieve the specified pH. Thereafter, an ultrasonic treatment was performed for 30 seconds. After homogenization (10000 rpm, 2 minutes), the absorbance (wavelength: 300 nm) of the supernatant at the time of standing-still at 50°C was measured, and the dispersibility in water was confirmed.
Any of the absorbance initial values was around 1.8, and the following evaluation was performed on absorbance measurement after 200 hours, after 224 hours, and 248 hours.

Evaluation criteria

5: The absorbance is 1.7 or more.
4: The absorbance is 1.5 or more and less than 1.7.
3: The absorbance is 1.3 or more and less than 1.5.
2: The absorbance is 1.0 or more and less than 1.3.
1: The absorbance is less than 1.0.

<Evaluation of feeling in use>

The formulation of dimethicone prepared in the emulsification stability confirmation test 1 was evaluated as follows. A sensory evaluation was performed by 5 panelists, and the average value was taken as the evaluation score.

(Evaluation item: freshness during spreading)

| | |
|---|---|
| Having much freshness | 5 |
| Having freshness | 4 |
| Having slight freshness | 3 |
| Scarcely having freshness | 2 |
| Having no freshness | 1 |

(Evaluation item: freedom from stickiness after spreading)

| | |
|---|---|
| Free from stickiness | 5 |
| Substantially free from stickiness | 4 |
| Slightly sticky | 3 |
| Sticky | 2 |
| Very sticky | 1 |

(Evaluation item: body feeling on application)

| | |
|---|---|
| Having much body feeling | 5 |
| Having body feeling | 4 |
| Having slight body feeling | 3 |
| Scarcely having body feeling | 2 |
| Having no body feeling | 1 |

(Evaluation item: extensibility during spreading)

| | |
|---|---|
| Excellently extensible | 5 |
| Well extensible | 4 |
| Slightly well extensible | 3 |
| Less extensible | 2 |
| Poorly extensible | 1 |

(Evaluation item: smoothness after spreading)

| | |
|---|---|
| Having much smoothness | 5 |
| Having smoothness | 4 |
| Having slight smoothness | 3 |
| Having insufficient smoothness | 2 |
| Having no smoothness | 1 |

The above four tests and evaluation, that is, the emulsification stability confirmation test 1, emulsification stability confirmation test 2, dispersibility stability confirmation test, and evaluation of feeling in use were performed using each of the compositions of Examples and Comparative Examples. The results are shown in Tables 1 to 5.

As described above, in the tables, "Component B proportion (%)" is one relative to the component A, "Component C mass (%)" is one relative to the total of the component A and the component C, "Component D mass(%)" is one relative to the total of the component A and the component D, and "Component E mass(%)" is one relative to the component A.

[Table 1-1]

| Number | Example 1 | | | Example 2 | | | Example 3 | | | Example 4 | | | Comparative Example 1 | | | Comparative Example 2 | | | Comparative Example 3 | | | Example 5 | | | Example 6 | | | Example 7 | | | Example 8 | | | Example 9 | | | Example 10 | | |
|---|---|---|---|---|---|---|---|---|---|---|---|---|---|---|---|---|---|---|---|---|---|---|---|---|---|---|---|---|---|---|---|---|---|---|---|---|---|---|---|
| Composition | | | | | | | | | | | | | | | | | | | | | | | | | | | | | | | | | | | | | | | |
| Component A | Yes | | | Yes | | | Yes | | | Yes | | | Yes | | | Yes | | | Yes | | | Yes | | | Yes | | | Yes | | | Yes | | | Yes | | | Yes | | |
| Component B proportion (%) | 0.032 | | | 1.33 | | | 0.312 | | | 6.85 | | | 0 | | | 0 | | | 0 | | | – | | | – | | | – | | | | | | – | | | – | | |
| Component C mass (%) | – | | | – | | | – | | | – | | | – | | | – | | | – | | | 1.3 | | | 5.1 | | | 6.5 | | | 5.8 | | | 1.3 | | | 1.3 | | |
| Component D mass (%) | – | | | – | | | – | | | – | | | – | | | – | | | – | | | 0.08 | | | 0.14 | | | 2.1 | | | 0.55 | | | 0.08 | | | 0.08 | | |
| Component E mass (%) | – | | | – | | | – | | | – | | | – | | | – | | | – | | | – | | | – | | | – | | | | | | – | | | – | | |
| Cocamide MEA | – | | | – | | | – | | | – | | | – | | | – | | | – | | | – | | | – | | | – | | | | | | – | | | – | | |
| Lauramide MEA | – | | | – | | | – | | | – | | | – | | | – | | | – | | | – | | | – | | | – | | | | | | – | | | – | | |
| Carbon atoms of R1 to R6 of components A to E | (A,11) (B,11) | | | (A,11) (B,11) | | | (A,11) (B,11) | | | (A,11) (B,11) | | | (A,11) | | | (A,11) | | | (A,11) | | | (A,11) (C,11) (D,11) | | | (A,11) (C,11) (D,11) | | | (A,11) (C,11) (D,11) | | | (A,11) (C,11) (D,11) | | | (A,11) (C,11) (D,11) | | | (A,11) (C,11) (D,11) | | |
| M1 to M7 of components A to D | Na | | | Na | | | Na | | | Na | | | Na | | | Na | | | Na | | | Na | | | Na | | | Na | | | Na | | | Na | | | Na | | |
| pH of mixed solution | 7 | | | 7 | | | 7 | | | 7 | | | 7 | | | 7 | | | 7 | | | 7 | | | 7 | | | 7 | | | 7 | | | 7 | | | 7 | | |
| Formulation evaluation | | | | | | | | | | | | | | | | | | | | | | | | | | | | | | | | | | | | | | | |
| pH | 7 | | | 7 | | | 7 | | | 7 | | | 7 | | | 7 | | | 7 | | | 7 | | | 7 | | | 7 | | | 7 | | | 7 | | | 7 | | |
| Emulsification stability confirmation test 1 | After 2.0 months | After 2.5 months | After 3.0 months | After 2.0 months | After 2.5 months | After 3.0 months | After 2.0 months | After 2.5 months | After 3.0 months | After 2.0 months | After 2.5 months | After 3.0 months | After 2.0 months | After 2.5 months | After 3.0 months | After 2.0 months | After 2.5 months | After 3.0 months | After 2.0 months | After 2.5 months | After 3.0 months | After 2.0 months | After 2.5 months | After 3.0 months | After 2.0 months | After 2.5 months | After 3.0 months | After 2.0 months | After 2.5 months | After 3.0 months | After 2.0 months | After 2.5 months | After 3.0 months | After 2.0 months | After 2.5 months | After 3.0 months | After 2.0 months | After 2.5 months | After 3.0 months |
| Ethylhexyl palmitate | 5 | 3 | 3 | 5 | 3 | 2 | 5 | 3 | 2 | 5 | 3 | 3 | 5 | 3 | 1 | 5 | 3 | 1 | 5 | 3 | 1 | 4 | 3 | 2 | 5 | 3 | 3 | 5 | 3 | 3 | 5 | 3 | 2 | 4 | 3 | 1 | 4 | 3 | 1 |
| Olive oil | 5 | 3 | 3 | 5 | 3 | 2 | 5 | 3 | 2 | 5 | 3 | 3 | 5 | 2 | 1 | 5 | 2 | 1 | 4 | 2 | 1 | 4 | 3 | 2 | 5 | 3 | 2 | 5 | 3 | 3 | 5 | 3 | 2 | 5 | 3 | 2 | 5 | 3 | 2 |
| Mineral oil | 5 | 3 | 3 | 5 | 3 | 2 | 5 | 3 | 3 | 5 | 3 | 2 | 5 | 3 | 1 | 5 | 3 | 1 | 5 | 2 | 1 | 5 | 3 | 3 | 5 | 3 | 2 | 5 | 3 | 2 | 5 | 3 | 2 | 4 | 3 | 3 | 5 | 3 | 3 |
| Cyclomethicone | 5 | 3 | 2 | 5 | 3 | 3 | 5 | 3 | 2 | 5 | 3 | 2 | 4 | 2 | 1 | 4 | 2 | 1 | 4 | 2 | 1 | 5 | 3 | 2 | 5 | 3 | 2 | 4 | 3 | 2 | 5 | 3 | 3 | 5 | 4 | 3 | 5 | 3 | 3 |
| Dimethicone | 5 | 3 | 2 | 5 | 3 | 3 | 5 | 3 | 2 | 5 | 3 | 2 | 4 | 2 | 1 | 5 | 2 | 1 | 4 | 2 | 1 | 5 | 3 | 3 | 5 | 3 | 2 | 4 | 3 | 2 | 5 | 3 | 3 | 5 | 3 | 3 | 4 | 3 | 3 |
| Emulsification stability confirmation test 2 | | | | | | | | | | | | | | | | | | | | | | | | | | | | | | | | | | | | | | | |
| Cetostearyl alcohol | 5 | 3 | 2 | 5 | 3 | 2 | 5 | 3 | 2 | 5 | 3 | 2 | 4 | 2 | 1 | 4 | 2 | 1 | 5 | 3 | 1 | 5 | 3 | 2 | 5 | 3 | 3 | 5 | 3 | 2 | 5 | 3 | 2 | 5 | 3 | 2 | 5 | 3 | 2 |
| Myristyl alcohol | 5 | 3 | 3 | 5 | 3 | 2 | 5 | 3 | 2 | 5 | 3 | 2 | 4 | 2 | 1 | 5 | 2 | 1 | 4 | 2 | 1 | 5 | 3 | 2 | 5 | 3 | 3 | 5 | 3 | 2 | 5 | 3 | 2 | 5 | 4 | 2 | 5 | 4 | 2 |
| Dispersion stability confirmation test | After 200 hours | After 224 hours | After 248 hours | After 200 hours | After 224 hours | After 248 hours | After 200 hours | After 224 hours | After 248 hours | After 200 hours | After 224 hours | After 248 hours | After 200 hours | After 224 hours | After 248 hours | After 200 hours | After 224 hours | After 248 hours | After 200 hours | After 224 hours | After 248 hours | After 200 hours | After 224 hours | After 248 hours | After 200 hours | After 224 hours | After 248 hours | After 200 hours | After 224 hours | After 248 hours | After 200 hours | After 224 hours | After 248 hours | After 200 hours | After 224 hours | After 248 hours | After 200 hours | After 224 hours | After 248 hours |
| | 5 | 3 | 2 | 5 | 3 | 2 | 4 | 4 | 3 | 4 | 3 | 2 | 4 | 2 | 1 | 3 | 1 | 1 | 4 | 2 | 1 | 5 | 3 | 1 | 5 | 3 | 2 | 5 | 3 | 2 | 5 | 4 | 2 | 5 | 3 | 1 | 5 | 3 | 1 |
| Sensory evaluation | | | | | | | | | | | | | | | | | | | | | | | | | | | | | | | | | | | | | | | |
| Freshness | 5 | | | 5 | | | 5 | | | 4 | | | 4 | | | 3 | | | 3 | | | 3 | | | 3 | | | 3 | | | 3 | | | 3 | | | 3 | | |
| Freedom from stickiness | 5 | | | 5 | | | 5 | | | 4 | | | 4 | | | 4 | | | 3 | | | 3 | | | 3 | | | 3 | | | 3 | | | 3 | | | 3 | | |
| Body feeling | 3 | | | 3 | | | 3 | | | 3 | | | 3 | | | 3 | | | 3 | | | 5 | | | 5 | | | 5 | | | 5 | | | 5 | | | 5 | | |
| Extensibility | 3 | | | 3 | | | 3 | | | 3 | | | 3 | | | 3 | | | 3 | | | 3 | | | 3 | | | 3 | | | 3 | | | 3 | | | 3 | | |
| Smoothness | 3 | | | 3 | | | 3 | | | 3 | | | 3 | | | 3 | | | 3 | | | 3 | | | 3 | | | 3 | | | 3 | | | 3 | | | 3 | | |

[Table 1-2]

| Number | Example 11 | | | Example 12 | | | Example 13 | | | Comparative Example 4 | | | Comparative Example 5 | | | Comparative Example 6 | | | Example 14 | | | Example 14 | | | Example 15 | | | Example 16 | | | Example 17 | | |
|---|---|---|---|---|---|---|---|---|---|---|---|---|---|---|---|---|---|---|---|---|---|---|---|---|---|---|---|---|---|---|---|---|---|
| Composition | | | | | | | | | | | | | | | | | | | | | | | | | | | | | | | | | |
| Component A | Yes | | | Yes | | | Yes | | | Yes | | | Yes | | | Yes | | | Yes | | | Yes | | | Yes | | | Yes | | | Yes | | |
| Component B proportion (%) | – | | | – | | | – | | | – | | | – | | | – | | | – | | | – | | | – | | | – | | | – | | |
| Component C mass (%) | – | | | – | | | – | | | – | | | – | | | – | | | – | | | – | | | – | | | – | | | – | | |
| Component D mass (%) | – | | | – | | | – | | | – | | | – | | | – | | | – | | | – | | | – | | | – | | | – | | |
| Component E mass (%) | 0.009 | | | 0.215 | | | 4.309 | | | 0 | | | 0 | | | 0 | | | 0.03 | | | 0.091 | | | 0.215 | | | 0.215 | | | 2.153 | | |
| Cocamide MEA | – | | | – | | | – | | | 0 | | | 0.064 | | | 0 | | | 0 | | | 0 | | | 0.064 | | | 0 | | | 0 | | |
| Lauramide MEA | – | | | – | | | – | | | 0 | | | 0 | | | 0.064 | | | 0 | | | 0 | | | 0 | | | 0.064 | | | 0 | | |
| Carbon atoms of R1 to R6 of components A to E | (A,11) (E,11) | | | (A,11) (E,11) | | | (A,11) (E,11) | | | (A,11) | | | (A,11) | | | (A,11) | | | (A,11) (E,11) | | | (A,11) (E,11) | | | (A,11) (E,11) | | | (A,11) (E,11) | | | (A,11) (E,11) | | |
| M1 to M7 of components A to D | Na | | | Na | | | Na | | | Na | | | Na | | | Na | | | Na | | | Na | | | Na | | | Na | | | Na | | |
| pH of mixed solution | 7 | | | 7 | | | 7 | | | 7 | | | 7 | | | 7 | | | 7 | | | 7 | | | 7 | | | 7 | | | 7 | | |
| Formulation evaluation | | | | | | | | | | | | | | | | | | | | | | | | | | | | | | | | | |
| pH | 7 | | | 7 | | | 7 | | | 7 | | | 7 | | | 7 | | | 7 | | | 7 | | | 7 | | | 7 | | | 7 | | |
| Emulsification stability confirmation test 1 | After 2.0 months | After 2.5 months | After 3.0 months | After 2.0 months | After 2.5 months | After 3.0 months | After 2.0 months | After 2.5 months | After 3.0 months | After 2.0 months | After 2.5 months | After 3.0 months | After 2.0 months | After 2.5 months | After 3.0 months | After 2.0 months | After 2.5 months | After 3.0 months | After 2.0 months | After 2.5 months | After 3.0 months | After 2.0 months | After 2.5 months | After 3.0 months | After 2.0 months | After 2.5 months | After 3.0 months | After 2.0 months | After 2.5 months | After 3.0 months | After 2.0 months | After 2.5 months | After 3.0 months |
| Ethylhexyl palmitate | 5 | 3 | 3 | 5 | 3 | 2 | 5 | 3 | 2 | 5 | 3 | 1 | 5 | 3 | 1 | 5 | 2 | 1 | 5 | 3 | 2 | 5 | 3 | 2 | 5 | 3 | 3 | 5 | 3 | 2 | 5 | 3 | 2 |
| Olive oil | 5 | 3 | 3 | 5 | 3 | 3 | 5 | 3 | 2 | 5 | 2 | 1 | 5 | 2 | 1 | 5 | 3 | 1 | 5 | 3 | 2 | 5 | 3 | 3 | 5 | 3 | 2 | 5 | 3 | 3 | 5 | 3 | 2 |
| Mineral oil | 5 | 3 | 3 | 5 | 3 | 2 | 5 | 3 | 2 | 5 | 2 | 1 | 5 | 3 | 1 | 5 | 3 | 1 | 5 | 3 | 3 | 5 | 3 | 2 | 5 | 3 | 2 | 5 | 3 | 2 | 5 | 3 | 3 |
| Cyclomethicone | 5 | 3 | 3 | 5 | 3 | 3 | 5 | 3 | 2 | 4 | 2 | 1 | 4 | 2 | 1 | 4 | 2 | 1 | 5 | 3 | 3 | 5 | 3 | 2 | 5 | 3 | 3 | 5 | 3 | 2 | 5 | 3 | 3 |
| Dimethicone | 5 | 3 | 2 | 5 | 3 | 3 | 5 | 3 | 2 | 5 | 3 | 1 | 4 | 2 | 1 | 5 | 2 | 1 | 5 | 3 | 2 | 5 | 3 | 2 | 5 | 3 | 2 | 5 | 3 | 2 | 5 | 3 | 2 |
| Emulsification stability confirmation test 2 | | | | | | | | | | | | | | | | | | | | | | | | | | | | | | | | | |
| Cetostearyl alcohol | 5 | 3 | 2 | 5 | 3 | 2 | 5 | 3 | 3 | 4 | 2 | 1 | 4 | 2 | 1 | 4 | 2 | 1 | 5 | 3 | 2 | 5 | 3 | 2 | 5 | 3 | 2 | 5 | 3 | 3 | 5 | 3 | 2 |
| Myristyl alcohol | 5 | 3 | 2 | 5 | 3 | 2 | 5 | 3 | 3 | 4 | 2 | 1 | 4 | 2 | 1 | 4 | 2 | 1 | 5 | 3 | 2 | 5 | 3 | 3 | 5 | 3 | 2 | 5 | 3 | 2 | 5 | 3 | 2 |
| Dispersion stability confirmation test | After 200 hours | After 224 hours | After 248 hours | After 200 hours | After 224 hours | After 248 hours | After 200 hours | After 224 hours | After 248 hours | After 200 hours | After 224 hours | After 248 hours | After 200 hours | After 224 hours | After 248 hours | After 200 hours | After 224 hours | After 248 hours | After 200 hours | After 224 hours | After 248 hours | After 200 hours | After 224 hours | After 248 hours | After 200 hours | After 224 hours | After 248 hours | After 200 hours | After 224 hours | After 248 hours | After 200 hours | After 224 hours | After 248 hours |
| | 4 | 2 | 2 | 5 | 3 | 3 | 4 | 3 | 2 | 3 | 2 | 1 | 3 | 1 | 1 | 3 | 1 | 1 | 4 | 3 | 1 | 5 | 3 | 2 | 5 | 3 | 2 | 5 | 3 | 2 | 5 | 3 | 2 |
| Sensory evaluation | | | | | | | | | | | | | | | | | | | | | | | | | | | | | | | | | |
| Freshness | 3 | | | 3 | | | 3 | | | 3 | | | 3 | | | 3 | | | 3 | | | 3 | | | 3 | | | 3 | | | 3 | | |
| Freedom from stickiness | 3 | | | 3 | | | 3 | | | 3 | | | 3 | | | 3 | | | 3 | | | 3 | | | 3 | | | 3 | | | 3 | | |
| Body feeling | 3 | | | 3 | | | 3 | | | 3 | | | 3 | | | 3 | | | 3 | | | 3 | | | 3 | | | 3 | | | 3 | | |
| Extensibility | 5 | | | 5 | | | 4 | | | 4 | | | 3 | | | 3 | | | 5 | | | 5 | | | 5 | | | 5 | | | 5 | | |
| Smoothness | 4 | | | 5 | | | 5 | | | 3 | | | 3 | | | 3 | | | 5 | | | 5 | | | 5 | | | 5 | | | 5 | | |

[Table 2-1]

| Number | Example 18 | | | Example 19 | | | Example 20 | | | Example 21 | | | Example 22 | | | Example 23 | | | Example 24 | | | Example 25 | | | Example 26 | | |
|---|---|---|---|---|---|---|---|---|---|---|---|---|---|---|---|---|---|---|---|---|---|---|---|---|---|---|---|
| Composition | | | | | | | | | | | | | | | | | | | | | | | | | | | |
| Component A | Yes | | | Yes | | | Yes | | | Yes | | | Yes | | | Yes | | | Yes | | | Yes | | | Yes | | |
| Component B proportion (%) | 0.032 | | | 1.33 | | | 6.85 | | | 0.032 | | | 1.33 | | | 6.85 | | | – | | | – | | | – | | |
| Component C mass (%) | – | | | – | | | – | | | 1.3 | | | 5.1 | | | 6.5 | | | 1.3 | | | 5.1 | | | 6.5 | | |
| Component D mass (%) | – | | | – | | | – | | | 0.08 | | | 0.14 | | | 2.1 | | | 0.08 | | | 0.14 | | | 2.1 | | |
| Component E mass (%) | 0.009 | | | 0.215 | | | 4.309 | | | – | | | – | | | – | | | 0.009 | | | 0.215 | | | 4.309 | | |
| Cocamide MEA | – | | | – | | | – | | | – | | | – | | | – | | | – | | | – | | | – | | |
| Lauramide MEA | – | | | – | | | – | | | – | | | – | | | – | | | – | | | – | | | – | | |
| Carbon atoms of R1 to R6 of components A to E | (A,11)<br>(B,11)<br>(E,11) | | | (A,11)<br>(B,11)<br>(E,11) | | | (A,11)<br>(B,11)<br>(E,11) | | | (A,11)<br>(B,11)<br>(C,11)<br>(D,11) | | | (A,11)<br>(B,11)<br>(C,11)<br>(D,11) | | | (A,11)<br>(B,11)<br>(C,11)<br>(D,11) | | | (A,11)<br>(C,11)<br>(D,11)<br>(E,11) | | | (A,11)<br>(C,11)<br>(D,11)<br>(E,11) | | | (A,11)<br>(C,11)<br>(D,11)<br>(E,11) | | |
| M1 to M7 of components A to D | Na | | | Na | | | Na | | | Na | | | Na | | | Na | | | Na | | | Na | | | Na | | |
| pH of mixed solution | 7 | | | 7 | | | 7 | | | 7 | | | 7 | | | 7 | | | 7 | | | 7 | | | 7 | | |
| Formulation evaluation | | | | | | | | | | | | | | | | | | | | | | | | | | | |
| pH | 7 | | | 7 | | | 7 | | | 7 | | | 7 | | | 7 | | | 7 | | | 7 | | | 7 | | |
| Emulsification stability confirmation test 1 | After 2.0 months | After 2.5 months | After 3.0 months | After 2.0 months | After 2.5 months | After 3.0 months | After 2.0 months | After 2.5 months | After 3.0 months | After 2.0 months | After 2.5 months | After 3.0 months | After 2.0 months | After 2.5 months | After 3.0 months | After 2.0 months | After 2.5 months | After 3.0 months | After 2.0 months | After 2.5 months | After 3.0 months | After 2.0 months | After 2.5 months | After 3.0 months | After 2.0 months | After 2.5 months | After 3.0 months |
| Ethylhexyl palmitate | 5 | 4 | 3 | 5 | 5 | 4 | 5 | 5 | 4 | 5 | 4 | 3 | 5 | 5 | 4 | 5 | 5 | 4 | 5 | 5 | 4 | 5 | 5 | 4 | 5 | 5 | 4 |
| Olive oil | 5 | 4 | 3 | 5 | 5 | 4 | 5 | 5 | 4 | 5 | 4 | 3 | 5 | 5 | 4 | 5 | 5 | 4 | 5 | 5 | 4 | 5 | 5 | 4 | 5 | 5 | 4 |
| Mineral oil | 5 | 5 | 4 | 5 | 5 | 4 | 5 | 5 | 4 | 5 | 4 | 4 | 5 | 5 | 4 | 5 | 5 | 4 | 5 | 5 | 4 | 5 | 5 | 4 | 5 | 5 | 4 |
| Cyclomethicone | 5 | 5 | 4 | 5 | 5 | 4 | 5 | 4 | 3 | 5 | 5 | 4 | 5 | 5 | 4 | 5 | 4 | 3 | 5 | 4 | 3 | 5 | 4 | 4 | 5 | 4 | 3 |
| Dimethicone | 5 | 5 | 4 | 5 | 5 | 4 | 5 | 4 | 3 | 5 | 5 | 4 | 5 | 5 | 4 | 5 | 4 | 3 | 5 | 4 | 3 | 5 | 5 | 4 | 5 | 4 | 3 |
| Emulsification stability confirmation test 2 | | | | | | | | | | | | | | | | | | | | | | | | | | | |
| Cetostearyl alcohol | 5 | 5 | 4 | 5 | 5 | 4 | 5 | 5 | 4 | 5 | 5 | 4 | 5 | 4 | 4 | 5 | 5 | 4 | 5 | 5 | 4 | 5 | 5 | 4 | 5 | 5 | 3 |
| Myristyl alcohol | 5 | 5 | 4 | 5 | 5 | 4 | 5 | 5 | 4 | 5 | 5 | 4 | 5 | 5 | 4 | 5 | 5 | 4 | 5 | 5 | 4 | 5 | 5 | 4 | 5 | 5 | 4 |
| Dispersion stability confirmation test | After 200 hours | After 224 hours | After 248 hours | After 200 hours | After 224 hours | After 248 hours | After 200 hours | After 224 hours | After 248 hours | After 200 hours | After 224 hours | After 248 hours | After 200 hours | After 224 hours | After 248 hours | After 200 hours | After 224 hours | After 248 hours | After 200 hours | After 224 hours | After 248 hours | After 200 hours | After 224 hours | After 248 hours | After 200 hours | After 224 hours | After 248 hours |
| | 5 | 5 | 3 | 5 | 5 | 3 | 5 | 5 | 2 | 5 | 5 | 3 | 5 | 5 | 3 | 5 | 5 | 2 | 5 | 5 | 3 | 5 | 5 | 3 | 5 | 5 | 1 |
| Sensory evaluation | | | | | | | | | | | | | | | | | | | | | | | | | | | |
| Freshness | 5 | | | 5 | | | 5 | | | 5 | | | 5 | | | 4 | | | 3 | | | 3 | | | 3 | | |
| Freedom from stickiness | 5 | | | 5 | | | 4 | | | 5 | | | 5 | | | 5 | | | 3 | | | 3 | | | 3 | | |
| Body feeling | 3 | | | 3 | | | 3 | | | 5 | | | 5 | | | 5 | | | 5 | | | 5 | | | 5 | | |
| Extensibility | 5 | | | 5 | | | 5 | | | 3 | | | 3 | | | 3 | | | 5 | | | 5 | | | 5 | | |
| Smoothness | 4 | | | 5 | | | 5 | | | 3 | | | 3 | | | 3 | | | 5 | | | 5 | | | 5 | | |

[Table 2-2]

| Number | Example 27 | | | Example 28 | | | Example 29 | | | Comparative Example 7 | | | Comparative Example 8 | | | Comparative Example 9 | | |
|---|---|---|---|---|---|---|---|---|---|---|---|---|---|---|---|---|---|---|
| Composition | | | | | | | | | | | | | | | | | | |
| Component A | Yes | | | Yes | | | Yes | | | No | | | No | | | No | | |
| Component B proportion (%) | 0.032 | | | 1.33 | | | 6.85 | | | 0.032 | | | 1.33 | | | 6.85 | | |
| Component C mass (%) | 1.3 | | | 5.1 | | | 6.5 | | | 1.3 | | | 5.1 | | | 6.5 | | |
| Component D mass (%) | 0.08 | | | 0.14 | | | 2.1 | | | 0.08 | | | 0.14 | | | 21 | | |
| Component E mass (%) | 0.009 | | | 0.215 | | | 4.309 | | | 0.009 | | | 0.215 | | | 4.309 | | |
| Cocamide MEA | - | | | - | | | - | | | - | | | - | | | - | | |
| Lauramide MEA | - | | | - | | | - | | | - | | | - | | | - | | |
| Carbon atoms of R1 to R6 of components A to E | (A,11) (B,11) (C,11) (D,11) (E,11) | | | (A,11) (B,11) (C,11) (D,11) (E,11) | | | (A,11) (B,11) (C,11) (D,11) (E,11) | | | (B,11) (C,11) (D,11) (E,11) | | | (B,11) (C,11) (D,11) (E,11) | | | (B,11) (C,11) (D,11) (E,11) | | |
| M1 to M7 of components A to D | Na | | | Na | | | Na | | | Na | | | Na | | | Na | | |
| pH of mixed solution | 7 | | | 7 | | | 7 | | | 7 | | | 7 | | | 7 | | |
| Formulation evaluation | | | | | | | | | | | | | | | | | | |
| pH | 7 | | | 7 | | | 7 | | | 7 | | | 7 | | | 7 | | |
| Emulsification stability confirmation test 1 | After 2.0 months | After 2.5 months | After 3.0 months | After 2.0 months | After 2.5 months | After 3.0 months | After 2.0 months | After 2.5 months | After 3.0 months | After 2.0 months | After 2.5 months | After 3.0 months | After 2.0 months | After 2.5 months | After 3.0 months | After 2.0 months | After 2.5 months | After 3.0 months |

(continued)

| Number | Example 27 | | | Example 28 | | | Example 29 | | | Comparative Example 7 | | | Comparative Example 8 | | | Comparative Example 9 | | |
|---|---|---|---|---|---|---|---|---|---|---|---|---|---|---|---|---|---|---|
| Ethylhexyl palmitate | 5 | 5 | 4 | 5 | 5 | 5 | 5 | 5 | 5 | 4 | 2 | 2 | 3 | 2 | 2 | 4 | 2 | 2 |
| Olive oil | 5 | 5 | 4 | 5 | 5 | 5 | 5 | 5 | 5 | 3 | 2 | 1 | 4 | 2 | 1 | 4 | 2 | 2 |
| Mineral oil | 5 | 5 | 5 | 5 | 5 | 5 | 5 | 5 | 5 | 3 | 2 | 2 | 3 | 2 | 2 | 4 | 2 | 2 |
| Cyclomethicone | 5 | 5 | 5 | 5 | 5 | 5 | 5 | 5 | 4 | 4 | 2 | 1 | 3 | 2 | 1 | 3 | 2 | 1 |
| Dimethicone | 5 | 5 | 5 | 5 | 5 | 5 | 5 | 5 | 4 | 3 | 2 | 2 | 4 | 2 | 2 | 3 | 2 | 1 |
| Emulsification stability confirmation test 2 | | | | | | | | | | | | | | | | | | |
| Cetostearyl alcohol | 5 | 5 | 5 | 5 | 5 | 5 | 5 | 5 | 5 | 3 | 2 | 1 | 3 | 1 | 1 | 3 | 2 | 1 |
| alcohol | 5 | 5 | 5 | 5 | 5 | 5 | 5 | 5 | 5 | 3 | 2 | 1 | 3 | 2 | 1 | 3 | 2 | 1 |
| Myristyl Dispersion stability confirmation test | After 200 hours | After 224 hours | After 248 hours | After 200 hours | After 224 hours | After 248 hours | After 200 hours | After 224 hours | After 248 hours | After 200 hours | After 224 hours | After 248 hours | After 200 hours | After 224 hours | After 248 hours | After 200 hours | After 224 hours | After 248 hours |
| | 5 | 5 | 5 | 5 | 5 | 5 | 5 | 5 | 4 | 4 | 1 | 1 | 3 | 1 | 1 | 3 | 1 | 1 |
| Sensory evaluation | | | | | | | | | | | | | | | | | | |
| Freshness | 5 | | | 5 | | | 5 | | | 3 | | | 3 | | | 2 | | |
| Freedom from stickiness | 5 | | | 5 | | | 4 | | | 2 | | | 2 | | | 3 | | |
| Body feeling | 5 | | | 5 | | | 5 | | | 3 | | | 2 | | | 3 | | |
| Extensibility | 5 | | | 5 | | | 5 | | | 2 | | | 3 | | | 2 | | |
| Smoothness | 5 | | | 5 | | | 5 | | | 3 | | | 3 | | | 2 | | |

[Table 3-1]

| Number | Example 30 | | | Example 31 | | | Example 32 | | | Comparative Example 10 | | | Comparative Example 11 | | | Example 33 | | | Example 34 | | | Example 35 | | | Comparative Example 12 | | | Comparative Example 13 | | |
|---|---|---|---|---|---|---|---|---|---|---|---|---|---|---|---|---|---|---|---|---|---|---|---|---|---|---|---|---|---|---|
| Composition | | | | | | | | | | | | | | | | | | | | | | | | | | | | | | |
| Component A | Yes | | | Yes | | | Yes | | | No | | | Yes | | | Yes | | | Yes | | | Yes | | | No | | | Yes | | |
| Component B proportion (%) | 0.032 | | | 1.33 | | | 6.85 | | | 1.33 | | | – | | | 0.032 | | | 1.33 | | | 6.85 | | | 1.33 | | | – | | |
| Component C mass (%) | 1.3 | | | 5.1 | | | 6.5 | | | 5.1 | | | – | | | 1.3 | | | 5.1 | | | 6.5 | | | 5.1 | | | – | | |
| Component D mass (%) | 0.08 | | | 0.14 | | | 2.1 | | | 0.14 | | | – | | | 0.08 | | | 0.14 | | | 2.1 | | | 0.14 | | | – | | |
| Component E mass (%) | 0.009 | | | 0.215 | | | 4.309 | | | 0.215 | | | – | | | 0.009 | | | 0.215 | | | 4.309 | | | 0.215 | | | – | | |
| Cocamide MEA | – | | | – | | | – | | | – | | | – | | | – | | | – | | | – | | | – | | | – | | |
| Lauramide MEA | – | | | – | | | – | | | – | | | – | | | – | | | – | | | – | | | – | | | – | | |
| Carbon atoms of R1 to R6 of components A to E | (A,7)<br>(B,7)<br>(C,7)<br>(D,7)<br>(E,7) | | | (A,7)<br>(B,7)<br>(C,7)<br>(D,7)<br>(E,7) | | | (A,7)<br>(B,7)<br>(C,7)<br>(D,7)<br>(E,7) | | | (B,7)<br>(C,7)<br>(D,7)<br>(E,7) | | | (A,7) | | | (A,13)<br>(B,13)<br>(C,13)<br>(D,13)<br>(E,13) | | | (A,13)<br>(B,13)<br>(C,13)<br>(D,13)<br>(E,13) | | | (A,13)<br>(B,13)<br>(C,13)<br>(D,13)<br>(E,13) | | | (B,13)<br>(C,13)<br>(D,13)<br>(E,13) | | | (A,13) | | |
| M1 to M7 of components A to D | Na | | | Na | | | Na | | | Na | | | Na | | | Na | | | Na | | | Na | | | Na | | | Na | | |
| pH of mixed solution | 7 | | | 7 | | | 7 | | | 7 | | | 7 | | | 7 | | | 7 | | | 7 | | | 7 | | | 7 | | |
| Formulation evaluation | | | | | | | | | | | | | | | | | | | | | | | | | | | | | | |
| pH | 7 | | | 7 | | | 7 | | | 7 | | | 7 | | | 7 | | | 7 | | | 7 | | | 7 | | | 7 | | |
| Emulsification stability confirmation test 1 | After 2.0 months | After 2.5 months | After 3.0 months | After 2.0 months | After 2.5 months | After 3.0 months | After 2.0 months | After 2.5 months | After 3.0 months | After 2.0 months | After 2.5 months | After 3.0 months | After 2.0 months | After 2.5 months | After 3.0 months | After 2.0 months | After 2.5 months | After 3.0 months | After 2.0 months | After 2.5 months | After 3.0 months | After 2.0 months | After 2.5 months | After 3.0 months | After 2.0 months | After 2.5 months | After 3.0 months | After 2.0 months | After 2.5 months | After 3.0 months |
| Ethylhexyl palmitate | 5 | 5 | 4 | 5 | 5 | 5 | 5 | 5 | 4 | 3 | 3 | 1 | 5 | 3 | 1 | 5 | 5 | 4 | 5 | 5 | 5 | 5 | 5 | 5 | 4 | 3 | 1 | 5 | 3 | 1 |
| Olive oil | 5 | 5 | 5 | 5 | 5 | 5 | 5 | 5 | 5 | 3 | 3 | 1 | 4 | 2 | 1 | 5 | 5 | 5 | 5 | 5 | 5 | 5 | 5 | 5 | 3 | 2 | 1 | 4 | 2 | 1 |
| Mineral oil | 5 | 5 | 5 | 5 | 5 | 5 | 5 | 5 | 5 | 3 | 2 | 1 | 5 | 3 | 1 | 5 | 5 | 5 | 5 | 5 | 5 | 5 | 5 | 5 | 4 | 2 | 1 | 5 | 3 | 1 |
| Cyclomethicone | 5 | 5 | 5 | 5 | 5 | 5 | 5 | 5 | 4 | 4 | 2 | 1 | 4 | 2 | 1 | 5 | 5 | 5 | 5 | 5 | 5 | 5 | 5 | 4 | 3 | 2 | 1 | 5 | 3 | 1 |
| Dimethicone | 5 | 5 | 4 | 5 | 5 | 5 | 5 | 5 | 4 | 4 | 2 | 1 | 3 | 2 | 1 | 5 | 5 | 5 | 5 | 5 | 5 | 5 | 5 | 4 | 4 | 2 | 1 | 3 | 2 | 1 |
| Emulsification stability confirmation test 2 | | | | | | | | | | | | | | | | | | | | | | | | | | | | | | |
| Cetostearyl alcohol | 5 | 5 | 5 | 5 | 5 | 5 | 5 | 5 | 5 | 4 | 3 | 1 | 4 | 2 | 1 | 5 | 5 | 5 | 5 | 5 | 5 | 5 | 5 | 5 | 3 | 2 | 1 | 4 | 2 | 1 |
| Myristyl alcohol | 5 | 5 | 5 | 5 | 5 | 5 | 5 | 5 | 5 | 4 | 2 | 1 | 4 | 2 | 1 | 5 | 5 | 5 | 5 | 5 | 5 | 5 | 5 | 5 | 3 | 2 | 1 | 4 | 2 | 1 |
| Dispersion stability confirmation test | After 200 hours | After 224 hours | After 248 hours | After 200 hours | After 224 hours | After 248 hours | After 200 hours | After 224 hours | After 248 hours | After 200 hours | After 224 hours | After 248 hours | After 200 hours | After 224 hours | After 248 hours | After 200 hours | After 224 hours | After 248 hours | After 200 hours | After 224 hours | After 248 hours | After 200 hours | After 224 hours | After 248 hours | After 200 hours | After 224 hours | After 248 hours | After 200 hours | After 224 hours | After 248 hours |
| | 5 | 5 | 5 | 5 | 5 | 5 | 5 | 5 | 4 | 3 | 2 | 1 | 3 | 1 | 1 | 5 | 5 | 5 | 5 | 5 | 5 | 5 | 5 | 5 | 3 | 2 | 1 | 3 | 1 | 1 |
| Sensory evaluation | | | | | | | | | | | | | | | | | | | | | | | | | | | | | | |
| Freshness | 5 | | | 5 | | | 5 | | | 3 | | | 3 | | | 5 | | | 5 | | | 4 | | | 3 | | | 3 | | |
| Freedom from stickiness | 5 | | | 5 | | | 4 | | | 1 | | | 2 | | | 5 | | | 5 | | | 5 | | | 2 | | | 3 | | |
| Body feeling | 5 | | | 5 | | | 5 | | | 2 | | | 2 | | | 5 | | | 5 | | | 5 | | | 2 | | | 2 | | |
| Extensibility | 5 | | | 5 | | | 5 | | | 2 | | | 2 | | | 5 | | | 5 | | | 5 | | | 2 | | | 2 | | |
| Smoothness | 4 | | | 5 | | | 5 | | | 2 | | | 3 | | | 4 | | | 5 | | | 5 | | | 3 | | | 3 | | |

[Table 3-2]

| Number | Example 36 | | | Example 37 | | | Example 38 | | | Comparative Example 14 | | | Comparative Example 15 | | | Example 39 | | | Example 40 | | | Example 41 | | | Example 42 | | | Example 43 | | | Example 44 | | |
|---|---|---|---|---|---|---|---|---|---|---|---|---|---|---|---|---|---|---|---|---|---|---|---|---|---|---|---|---|---|---|---|---|---|
| Composition | | | | | | | | | | | | | | | | | | | | | | | | | | | | | | | | | | |
| Component A | Yes | | | Yes | | | Yes | | | No | | | Yes | | | Yes | | | Yes | | | Yes | | | Yes | | | Yes | | | Yes | | |
| Component B proportion (%) | 0.032 | | | 1.33 | | | 6.85 | | | 1.33 | | | – | | | – | | | – | | | – | | | 0.032 | | | 1.33 | | | 6.85 | | |
| Component C mass (%) | 1.3 | | | 5.1 | | | 6.5 | | | 5.1 | | | – | | | – | | | – | | | – | | | – | | | – | | | – | | |
| Component D mass (%) | 0.08 | | | 0.14 | | | 2.1 | | | 0.14 | | | – | | | – | | | – | | | – | | | – | | | – | | | – | | |
| Component E mass (%) | 0.009 | | | 0.215 | | | 4.309 | | | 0.215 | | | – | | | 0.009 | | | 0.215 | | | 4.309 | | | 0.009 | | | 0.215 | | | 4.309 | | |
| Cocamide MEA | – | | | – | | | – | | | – | | | – | | | – | | | – | | | – | | | – | | | – | | | – | | |
| Lauramide MEA | – | | | – | | | – | | | – | | | – | | | – | | | – | | | – | | | – | | | – | | | – | | |
| Carbon atoms of R1 to R6 of components A to E | (A,15)<br>(B,15)<br>(C,15)<br>(D,15)<br>(E,15) | | | (A,15)<br>(B,15)<br>(C,15)<br>(D,15)<br>(E,15) | | | (A,15)<br>(B,15)<br>(C,15)<br>(D,15)<br>(E,15) | | | (B,15)<br>(C,15)<br>(D,15)<br>(E,15) | | | (A,15) | | | (A,17)<br>(E,17) | | | (A,17)<br>(E,17) | | | (A,17)<br>(E,17) | | | (A,11)<br>(B,11)<br>(E,17) | | | (A,11)<br>(B,11)<br>(E,17) | | | (A,11)<br>(B,11)<br>(E,17) | | |
| M1 to M7 of components A to D | Na | | | Na | | | Na | | | Na | | | Na | | | Na | | | Na | | | Na | | | Na | | | Na | | | Na | | |
| pH of mixed solution | 7 | | | 7 | | | 7 | | | 7 | | | 7 | | | 7 | | | 7 | | | 7 | | | 7 | | | 7 | | | 7 | | |
| Formulation evaluation | | | | | | | | | | | | | | | | | | | | | | | | | | | | | | | | | | |
| pH | 7 | | | 7 | | | 7 | | | 7 | | | 7 | | | 7 | | | 7 | | | 7 | | | 7 | | | 7 | | | 7 | | |
| Emulsification stability confirmation test 1 | After 2.0 months | After 2.5 months | After 3.0 months | After 2.0 months | After 2.5 months | After 3.0 months | After 2.0 months | After 2.5 months | After 3.0 months | After 2.0 months | After 2.5 months | After 3.0 months | After 2.0 months | After 2.5 months | After 3.0 months | After 2.0 months | After 2.5 months | After 3.0 months | After 2.0 months | After 2.5 months | After 3.0 months | After 2.0 months | After 2.5 months | After 3.0 months | After 2.0 months | After 2.5 months | After 3.0 months | After 2.0 months | After 2.5 months | After 3.0 months | After 2.0 months | After 2.5 months | After 3.0 months |
| Ethylhexyl palmitate | 5 | 5 | 5 | 5 | 5 | 5 | 5 | 5 | 5 | 4 | 2 | 1 | 5 | 3 | 1 | 5 | 3 | 3 | 5 | 3 | 2 | 5 | 4 | 2 | 5 | 5 | 4 | 5 | 5 | 3 | 5 | 5 | 4 |
| Olive oil | 5 | 5 | 4 | 5 | 5 | 5 | 5 | 5 | 5 | 3 | 1 | 1 | 4 | 2 | 1 | 5 | 3 | 3 | 5 | 4 | 3 | 5 | 3 | 2 | 5 | 5 | 5 | 5 | 5 | 4 | 5 | 5 | 4 |
| Mineral oil | 5 | 5 | 5 | 5 | 5 | 5 | 5 | 5 | 5 | 4 | 2 | 1 | 5 | 3 | 1 | 5 | 3 | 2 | 5 | 3 | 2 | 5 | 3 | 2 | 5 | 5 | 4 | 5 | 5 | 4 | 5 | 5 | 3 |
| Cyclomethicone | 5 | 5 | 5 | 5 | 5 | 5 | 5 | 5 | 5 | 3 | 1 | 1 | 5 | 3 | 1 | 5 | 3 | 3 | 5 | 3 | 3 | 5 | 3 | 2 | 5 | 4 | 3 | 5 | 5 | 3 | 5 | 4 | 3 |
| Dimethicone | 5 | 5 | 5 | 5 | 5 | 5 | 5 | 5 | 4 | 4 | 2 | 1 | 4 | 2 | 1 | 5 | 4 | 2 | 5 | 3 | 3 | 5 | 3 | 2 | 5 | 5 | 4 | 5 | 5 | 3 | 5 | 5 | 3 |
| Emulsification stability confirmation test 2 | | | | | | | | | | | | | | | | | | | | | | | | | | | | | | | | | | |
| Cetostearyl alcohol | 5 | 5 | 5 | 5 | 5 | 5 | 5 | 5 | 4 | 3 | 2 | 1 | 4 | 2 | 1 | 5 | 3 | 2 | 5 | 3 | 2 | 5 | 3 | 3 | 5 | 5 | 3 | 5 | 5 | 4 | 5 | 5 | 4 |
| Myristyl alcohol | 5 | 5 | 5 | 5 | 5 | 5 | 5 | 5 | 5 | 3 | 2 | 1 | 5 | 3 | 1 | 5 | 3 | 2 | 5 | 3 | 2 | 5 | 3 | 2 | 5 | 5 | 3 | 5 | 5 | 4 | 5 | 5 | 4 |
| Dispersion stability confirmation test | After 200 hours | After 224 hours | After 248 hours | After 200 hours | After 224 hours | After 248 hours | After 200 hours | After 224 hours | After 248 hours | After 200 hours | After 224 hours | After 248 hours | After 200 hours | After 224 hours | After 248 hours | After 200 hours | After 224 hours | After 248 hours | After 200 hours | After 224 hours | After 248 hours | After 200 hours | After 224 hours | After 248 hours | After 200 hours | After 224 hours | After 248 hours | After 200 hours | After 224 hours | After 248 hours | After 200 hours | After 224 hours | After 248 hours |
| | 5 | 5 | 5 | 5 | 5 | 5 | 5 | 5 | 5 | 3 | 2 | 1 | 4 | 1 | 1 | 5 | 3 | 2 | 5 | 4 | 3 | 4 | 3 | 2 | 5 | 5 | 3 | 5 | 5 | 4 | 5 | 4 | 2 |
| Sensory evaluation | | | | | | | | | | | | | | | | | | | | | | | | | | | | | | | | | | |
| Freshness | 5 | | | 5 | | | 5 | | | 2 | | | 2 | | | 3 | | | 3 | | | 3 | | | 5 | | | 5 | | | 5 | | |
| Freedom from stickiness | 5 | | | 5 | | | 5 | | | 1 | | | 3 | | | 3 | | | 3 | | | 3 | | | 5 | | | 5 | | | 5 | | |
| Body feeling | 5 | | | 5 | | | 5 | | | 2 | | | 2 | | | 3 | | | 3 | | | 3 | | | 5 | | | 5 | | | 5 | | |
| Extensibility | 5 | | | 5 | | | 4 | | | 2 | | | 2 | | | 5 | | | 5 | | | 4 | | | 5 | | | 5 | | | 5 | | |
| Smoothness | 4 | | | 5 | | | 5 | | | 3 | | | 3 | | | 4 | | | 5 | | | 5 | | | 4 | | | 5 | | | 5 | | |

[Table 3-3]

| Number | Example 45 | Example 46 | Example 47 | Comparative | Comparative | Example 48 | Example 49 | Example 50 | Example 51 | Example 52 | Example 53 | Comparative | Comparative |
|---|---|---|---|---|---|---|---|---|---|---|---|---|---|

| | | | | | | | | | | Example 16 | | | Example 17 | | | | | | | | | | | | | | | | | | | | | Example 18 | | | Example 19 | | |
|---|---|---|---|---|---|---|---|---|---|---|---|---|---|---|---|---|---|---|---|---|---|---|---|---|---|---|---|---|---|---|---|---|---|---|---|---|---|---|---|
| Composition | | | | | | | | | | | | | | | | | | | | | | | | | | | | | | | | | | | | | | | |
| Component A | Yes | | | Yes | | | Yes | | | No | | | Yes | | | Yes | | | Yes | | | Yes | | | Yes | | | Yes | | | Yes | | | No | | | Yes | | |
| Component B proportion (%) | 0.032 | | | 1.33 | | | 6.85 | | | 1.33 | | | – | | | 0.032 | | | 1.33 | | | 6.85 | | | 0.032 | | | 1.33 | | | 6.85 | | | 1.33 | | | – | | |
| Component C mass (%) | 1.3 | | | 5.1 | | | 6.5 | | | 5.1 | | | – | | | – | | | – | | | – | | | 1.3 | | | 5.1 | | | 6.5 | | | 5.1 | | | – | | |
| Component D mass(%) | 0.08 | | | 0.14 | | | 2.1 | | | 0.14 | | | – | | | – | | | – | | | – | | | 0.08 | | | 0.14 | | | 2.1 | | | 0.14 | | | – | | |
| Component E mass(%) | 0.009 | | | 0.215 | | | 4.309 | | | 0.215 | | | – | | | 0.009 | | | 0.215 | | | 4.309 | | | 0.009 | | | 0.215 | | | 4.309 | | | 0.215 | | | – | | |
| Cocamide MEA | – | | | – | | | – | | | – | | | – | | | – | | | – | | | – | | | – | | | – | | | – | | | – | | | – | | |
| Lauramide MEA | – | | | – | | | – | | | – | | | – | | | – | | | – | | | – | | | – | | | – | | | – | | | – | | | – | | |
| Carbon atoms of R1 to R6 of components A to E | (A,17)<br>(B,17)<br>(C,17)<br>(D,17)<br>(E,17) | | | (A,17)<br>(B,17)<br>(C,17)<br>(D,17)<br>(E,17) | | | (A,17)<br>(B,17)<br>(C,17)<br>(D,17)<br>(E,17) | | | (B,17)<br>(C,17)<br>(D,17)<br>(E,17) | | | (A,17) | | | (A,11)<br>(B,11)<br>(E,21) | | | (A,11)<br>(B,11)<br>(E,21) | | | (A,11)<br>(B,11)<br>(E,21) | | | (A,21)<br>(B,21)<br>(C,21)<br>(D,21)<br>(E,21) | | | (A,21)<br>(B,21)<br>(C,21)<br>(D,21)<br>(E,21) | | | (A,21)<br>(B,21)<br>(C,21)<br>(D,21)<br>(E,21) | | | (B,21)<br>(C,21)<br>(D,21)<br>(E,21) | | | (A,21) | | |
| M1 to M7 of components A to D | Na | | | Na | | | Na | | | Na | | | Na | | | Na | | | Na | | | Na | | | Na | | | Na | | | Na | | | Na | | | Na | | |
| pH of mixed solution | 7 | | | 7 | | | 7 | | | 7 | | | 7 | | | 7 | | | 7 | | | 7 | | | 7 | | | 7 | | | 7 | | | 7 | | | 7 | | |
| Formulation evaluation | | | | | | | | | | | | | | | | | | | | | | | | | | | | | | | | | | | | | | | |
| pH | 7 | | | 7 | | | 7 | | | 7 | | | 7 | | | 7 | | | 7 | | | 7 | | | 7 | | | 7 | | | 7 | | | 7 | | | 7 | | |
| Emulsification stability confirmation test 1 | After 2.0 months | After 2.5 months | After 3.0 months | After 2.0 months | After 2.5 months | After 3.0 months | After 2.0 months | After 2.5 months | After 3.0 months | After 2.0 months | After 2.5 months | After 3.0 months | After 2.0 months | After 2.5 months | After 3.0 months | After 2.0 months | After 2.5 months | After 3.0 months | After 2.0 months | After 2.5 months | After 3.0 months | After 2.0 months | After 2.5 months | After 3.0 months | After 2.0 months | After 2.5 months | After 3.0 months | After 2.0 months | After 2.5 months | After 3.0 months | After 2.0 months | After 2.5 months | After 3.0 months | After 2.0 months | After 2.5 months | After 3.0 months | After 2.0 months | After 2.5 months | After 3.0 months |
| Ethylhexyl palmitate | 5 | 5 | 5 | 5 | 5 | 5 | 5 | 5 | 5 | 3 | 2 | 1 | 4 | 2 | 1 | 5 | 4 | 3 | 5 | 5 | 4 | 5 | 5 | 4 | 5 | 5 | 4 | 5 | 5 | 5 | 5 | 5 | 4 | 3 | 2 | 1 | 4 | 2 | 1 |
| Olive oil | 5 | 5 | 4 | 5 | 5 | 5 | 5 | 5 | 5 | 3 | 2 | 1 | 4 | 2 | 1 | 5 | 5 | 5 | 5 | 5 | 4 | 5 | 5 | 4 | 5 | 5 | 5 | 5 | 5 | 5 | 5 | 5 | 5 | 3 | 1 | 1 | 4 | 2 | 1 |
| Mineral oil | 5 | 5 | 5 | 5 | 5 | 5 | 5 | 5 | 5 | 4 | 2 | 1 | 5 | 3 | 1 | 5 | 5 | 4 | 5 | 5 | 4 | 5 | 4 | 3 | 5 | 5 | 5 | 5 | 5 | 5 | 5 | 5 | 5 | 3 | 2 | 1 | 5 | 3 | 1 |
| Cyclomethicone | 5 | 5 | 5 | 5 | 5 | 5 | 5 | 5 | 4 | 3 | 1 | 1 | 3 | 1 | 1 | 5 | 4 | 3 | 5 | 5 | 4 | 5 | 4 | 3 | 5 | 5 | 5 | 5 | 5 | 5 | 5 | 5 | 4 | 4 | 2 | 1 | 4 | 2 | 1 |
| Dimethicone | 5 | 5 | 4 | 5 | 5 | 5 | 5 | 5 | 4 | 2 | 2 | 1 | 4 | 2 | 1 | 5 | 5 | 4 | 5 | 5 | 3 | 5 | 5 | 3 | 5 | 5 | 4 | 5 | 5 | 5 | 5 | 5 | 5 | 4 | 2 | 1 | 5 | 3 | 1 |
| Emulsification stability confirmation test 2 | | | | | | | | | | | | | | | | | | | | | | | | | | | | | | | | | | | | | | | |
| Cetostearyl alcohol | 5 | 5 | 5 | 5 | 5 | 5 | 5 | 5 | 5 | 4 | 3 | 1 | 4 | 2 | 1 | 5 | 5 | 4 | 5 | 5 | 4 | 5 | 5 | 4 | 5 | 5 | 5 | 5 | 5 | 5 | 5 | 5 | 4 | 4 | 3 | 1 | 4 | 2 | 1 |
| Myristyl alcohol | 5 | 5 | 5 | 5 | 5 | 5 | 5 | 5 | 5 | 3 | 2 | 1 | 5 | 3 | 1 | 5 | 5 | 4 | 5 | 5 | 4 | 5 | 5 | 4 | 5 | 5 | 5 | 5 | 5 | 5 | 5 | 5 | 5 | 3 | 2 | 1 | 4 | 2 | 1 |
| Dispersion stability confirmation test | After 200 hours | After 224 hours | After 248 hours | After 200 hours | After 224 hours | After 248 hours | After 200 hours | After 224 hours | After 248 hours | After 200 hours | After 224 hours | After 248 hours | After 200 hours | After 224 hours | After 248 hours | After 200 hours | After 224 hours | After 248 hours | After 200 hours | After 224 hours | After 248 hours | After 200 hours | After 224 hours | After 248 hours | After 200 hours | After 224 hours | After 248 hours | After 200 hours | After 224 hours | After 248 hours | After 200 hours | After 224 hours | After 248 hours | After 200 hours | After 224 hours | After 248 hours | After 200 hours | After 224 hours | After 248 hours |
| | 5 | 5 | 5 | 5 | 5 | 5 | 5 | 5 | 5 | 3 | 2 | 1 | 3 | 2 | 1 | 5 | 5 | 3 | 5 | 5 | 4 | 5 | 5 | 2 | 5 | 5 | 5 | 5 | 5 | 5 | 5 | 5 | 4 | 3 | 2 | 1 | 4 | 2 | 1 |
| Sensory evaluation | | | | | | | | | | | | | | | | | | | | | | | | | | | | | | | | | | | | | | | |
| Freshness | 5 | | | 5 | | | 5 | | | 3 | | | 2 | | | 5 | | | 5 | | | 5 | | | 5 | | | 5 | | | 5 | | | 2 | | | 3 | | |
| Freedom from stickiness | 5 | | | 5 | | | 4 | | | 1 | | | 3 | | | 5 | | | 5 | | | 5 | | | 5 | | | 5 | | | 4 | | | 1 | | | 2 | | |
| Body feeling | 5 | | | 5 | | | 5 | | | 2 | | | 2 | | | 5 | | | 5 | | | 5 | | | 5 | | | 5 | | | 5 | | | 2 | | | 2 | | |
| Extensibility | 5 | | | 5 | | | 5 | | | 2 | | | 3 | | | 5 | | | 5 | | | 5 | | | 5 | | | 5 | | | 5 | | | 3 | | | 3 | | |
| Smoothness | 5 | | | 5 | | | 5 | | | 3 | | | 2 | | | 5 | | | 5 | | | 5 | | | 5 | | | 5 | | | 5 | | | 2 | | | 3 | | |

[Table 4-1]

| Number | Example 54 | | | Example 55 | | | Example 56 | | | Comparative Example 20 | | | Comparative Example 21 | | | Example 57 | | | Example 58 | | | Example 59 | | | Comparative Example 22 | | | Comparative Example 23 | | |
|---|---|---|---|---|---|---|---|---|---|---|---|---|---|---|---|---|---|---|---|---|---|---|---|---|---|---|---|---|---|---|
| Composition | | | | | | | | | | | | | | | | | | | | | | | | | | | | | | |
| Component A | Yes | | | Yes | | | Yes | | | No | | | Yes | | | Yes | | | Yes | | | Yes | | | No | | | Yes | | |
| Component B proportion (%) | 0.032 | | | 1.33 | | | 6.85 | | | 1.33 | | | – | | | 0.032 | | | 1.33 | | | 6.85 | | | 1.33 | | | – | | |
| Component C mass (%) | 1.3 | | | 5.1 | | | 6.5 | | | 5.1 | | | – | | | 1.3 | | | 5.1 | | | 6.5 | | | 5.1 | | | – | | |
| Component D mass (%) | 0.08 | | | 0.14 | | | 2.1 | | | 0.14 | | | – | | | 0.08 | | | 0.14 | | | 2.1 | | | 0.14 | | | – | | |
| Component E mass (%) | 0.009 | | | 0.215 | | | 4.309 | | | 0.215 | | | – | | | 0.009 | | | 0.215 | | | 4.309 | | | 0.215 | | | – | | |
| Cocamide MEA | – | | | – | | | – | | | – | | | – | | | – | | | – | | | – | | | – | | | – | | |
| Lauramide MEA | – | | | – | | | – | | | – | | | – | | | – | | | – | | | – | | | – | | | – | | |
| Carbon atoms of R1 to R6 of components A to E | (A,11)<br>(B,11)<br>(C,11)<br>(D,11)<br>(E,11) | | | (A,11)<br>(B,11)<br>(C,11)<br>(D,11)<br>(E,11) | | | (A,11)<br>(B,11)<br>(C,11)<br>(D,11)<br>(E,11) | | | (B,11)<br>(C,11)<br>(D,11)<br>(E,11) | | | (A,11) | | | (A,11)<br>(B,11)<br>(C,11)<br>(D,11)<br>(E,11) | | | (A,11)<br>(B,11)<br>(C,11)<br>(D,11)<br>(E,11) | | | (A,11)<br>(B,11)<br>(C,11)<br>(D,11)<br>(E,11) | | | (B,11)<br>(C,11)<br>(D,11)<br>(E,11) | | | (A,11) | | |
| M1 to M7 of components A to D | K | | | K | | | K | | | K | | | K | | | TEA | | | TEA | | | TEA | | | TEA | | | TEA | | |
| pH of mixed solution | 7 | | | 7 | | | 7 | | | 7 | | | 7 | | | 7 | | | 7 | | | 7 | | | 7 | | | 7 | | |
| Formulation evaluation | | | | | | | | | | | | | | | | | | | | | | | | | | | | | | |
| pH | 7 | | | 7 | | | 7 | | | 7 | | | 7 | | | 7 | | | 7 | | | 7 | | | 7 | | | 7 | | |
| Emulsification stability confirmation test 1 | After 2.0 months | After 2.5 months | After 3.0 months | After 2.0 months | After 2.5 months | After 3.0 months | After 2.0 months | After 2.5 months | After 3.0 months | After 2.0 months | After 2.5 months | After 3.0 months | After 2.0 months | After 2.5 months | After 3.0 months | After 2.0 months | After 2.5 months | After 3.0 months | After 2.0 months | After 2.5 months | After 3.0 months | After 2.0 months | After 2.5 months | After 3.0 months | After 2.0 months | After 2.5 months | After 3.0 months | After 2.0 months | After 2.5 months | After 3.0 months |
| Ethylhexyl palmitate | 5 | 5 | 4 | 5 | 5 | 5 | 5 | 5 | 5 | 3 | 2 | 1 | 4 | 2 | 1 | 5 | 5 | 5 | 5 | 5 | 5 | 5 | 5 | 5 | 4 | 3 | 1 | 4 | 2 | 1 |
| Olive oil | 5 | 5 | 4 | 5 | 5 | 5 | 5 | 5 | 5 | 3 | 1 | 1 | 4 | 2 | 1 | 5 | 5 | 4 | 5 | 5 | 5 | 5 | 5 | 5 | 3 | 3 | 1 | 4 | 2 | 1 |
| Mineral oil | 5 | 5 | 5 | 5 | 5 | 5 | 5 | 5 | 4 | 4 | 3 | 1 | 5 | 3 | 1 | 5 | 5 | 5 | 5 | 5 | 5 | 5 | 5 | 5 | 4 | 3 | 1 | 5 | 3 | 1 |
| Cyclomethicone | 5 | 5 | 5 | 5 | 5 | 5 | 5 | 5 | 4 | 4 | 2 | 1 | 4 | 2 | 1 | 5 | 5 | 5 | 5 | 5 | 5 | 5 | 5 | 4 | 3 | 2 | 1 | 4 | 2 | 1 |
| Dimethicone | 5 | 5 | 5 | 5 | 5 | 5 | 5 | 5 | 5 | 3 | 2 | 1 | 5 | 3 | 1 | 5 | 5 | 4 | 5 | 5 | 5 | 5 | 5 | 5 | 3 | 2 | 1 | 4 | 2 | 1 |
| Emulsification stability confirmation test 2 | | | | | | | | | | | | | | | | | | | | | | | | | | | | | | |
| Cetostearyl alcohol | 5 | 5 | 5 | 5 | 5 | 5 | 5 | 5 | 5 | 3 | 2 | 1 | 4 | 2 | 1 | 5 | 5 | 5 | 5 | 5 | 5 | 5 | 5 | 5 | 3 | 2 | 1 | 4 | 2 | 1 |
| Myristyl alcohol | 5 | 5 | 5 | 5 | 5 | 5 | 5 | 5 | 5 | 3 | 2 | 1 | 4 | 2 | 1 | 5 | 5 | 5 | 5 | 5 | 5 | 5 | 5 | 4 | 3 | 2 | 1 | 4 | 2 | 1 |
| Dispersion stability confirmation test | After 200 hours | After 224 hours | After 248 hours | After 200 hours | After 224 hours | After 248 hours | After 200 hours | After 224 hours | After 248 hours | After 200 hours | After 224 hours | After 248 hours | After 200 hours | After 224 hours | After 248 hours | After 200 hours | After 224 hours | After 248 hours | After 200 hours | After 224 hours | After 248 hours | After 200 hours | After 224 hours | After 248 hours | After 200 hours | After 224 hours | After 248 hours | After 200 hours | After 224 hours | After 248 hours |
| | 5 | 5 | 5 | 5 | 5 | 5 | 5 | 5 | 5 | 3 | 2 | 1 | 3 | 2 | 1 | 5 | 5 | 5 | 5 | 5 | 5 | 5 | 5 | 4 | 3 | 2 | 1 | 5 | 2 | 1 |
| Sensory evaluation | | | | | | | | | | | | | | | | | | | | | | | | | | | | | | |
| Freshness | 5 | | | 5 | | | 5 | | | 3 | | | 3 | | | 5 | | | 5 | | | 5 | | | 3 | | | 3 | | |
| Freedom from stickiness | 5 | | | 5 | | | 4 | | | 1 | | | 2 | | | 5 | | | 5 | | | 5 | | | 2 | | | 2 | | |
| Body feeling | 5 | | | 5 | | | 5 | | | 3 | | | 2 | | | 5 | | | 5 | | | 5 | | | 3 | | | 3 | | |
| Extensibility | 5 | | | 5 | | | 5 | | | 2 | | | 3 | | | 5 | | | 5 | | | 4 | | | 2 | | | 3 | | |
| Smoothness | 5 | | | 5 | | | 5 | | | 2 | | | 2 | | | 4 | | | 5 | | | 5 | | | 1 | | | 3 | | |

[Table 4-2]

| Number | Example 60 | | | Example 61 | | | Example 62 | | | Comparative Example 24 | | | Comparative Example 25 | | |
|---|---|---|---|---|---|---|---|---|---|---|---|---|---|---|---|
| Composition | | | | | | | | | | | | | | | |
| Component A | Yes | | | Yes | | | Yes | | | No | | | Yes | | |
| Component B proportion (%) | 0.032 | | | 1.33 | | | 6.85 | | | 1.33 | | | - | | |
| Component C mass (%) | 1.3 | | | 5.1 | | | 6.5 | | | 5.1 | | | - | | |
| Component D mass (%) | 0.08 | | | 0.14 | | | 2.1 | | | 0.14 | | | - | | |
| Component E mass (%) | 0.009 | | | 0.215 | | | 4.309 | | | 0.215 | | | - | | |
| Cocamide MEA | - | | | - | | | - | | | - | | | - | | |
| Lauramide MEA | - | | | - | | | - | | | - | | | - | | |
| Carbon atoms of R1 to R6 of components A to E | (A,11)<br>(B,11)<br>(C,11)<br>(D,11)<br>(E,11) | | | (A,11)<br>(B,11)<br>(C,11)<br>(D,11)<br>(E,11) | | | (A,11)<br>(B,11)<br>(C,11)<br>(D,11)<br>(E,11) | | | (B,11)<br>(C,11)<br>(D,11)<br>(E,11) | | | (A,11) | | |
| M1 to M7 of components A to D | Arg | | | Arg | | | Arg | | | Arg | | | Arg | | |
| pH of mixed solution | 7 | | | 7 | | | 7 | | | 7 | | | 7 | | |
| Formulation evaluation | | | | | | | | | | | | | | | |
| pH | 7 | | | 7 | | | 7 | | | 7 | | | 7 | | |
| Emulsification stability confirmation test 1 | After 20 months | After 2.5 months | After 3.0 months | After 2.0 months | After 2.5 months | After 3.0 months | After 2.0 months | After 2.5 months | After 3.0 months0 s | After 2.0 months | After 2.5 months | After 3.0 months | After 2.0 months | After 2.5 months | After 3.0 months |

(continued)

| Formulation evaluation | | | | | | | | | | | | | | | | |
|---|---|---|---|---|---|---|---|---|---|---|---|---|---|---|---|---|
| | Ethvlhexvl palmitate | 5 | 5 | 5 | 5 | 5 | 5 | 5 | 5 | 5 | 4 | 3 | 1 | 4 | 2 | 1 |
| | Olive oil | 5 | 5 | 5 | 5 | 5 | 5 | 5 | 5 | 5 | 3 | 2 | 1 | 5 | 3 | 1 |
| | Mineral oil | 5 | 5 | 5 | 5 | 5 | 5 | 5 | 5 | 5 | 3 | 2 | 1 | 4 | 2 | 1 |
| | Cyclomethicone | 5 | 5 | 4 | 5 | 5 | 5 | 5 | 5 | 5 | 3 | 2 | 1 | 4 | 2 | 1 |
| | Dimethicone | 5 | 5 | 4 | 5 | 5 | 5 | 5 | 5 | 4 | 3 | 2 | 1 | 4 | 2 | 1 |
| Emulsification stability confirmation test 2 | | | | | | | | | | | | | | | | |
| | Cetostearyl alcohol | 5 | 5 | 5 | 5 | 5 | 5 | 5 | 5 | 4 | 3 | 2 | 1 | 4 | 2 | 1 |
| | Mvristvl alcohol | 5 | 5 | 5 | 5 | 5 | 5 | 5 | 5 | 5 | 3 | 2 | 1 | 4 | 2 | 1 |
| Dispersion stability confirmation test | | After 200 hours | After 224 hours | After 248 hours | After 200 hours | After 224 hours | After 248 hours | After 200 hours | After 224 hours | After 24 hours 48 | After 200 hours | After 224 hours | After 248 hours | After 200 hours | After 224 hours | After 248 hours |
| | | 5 | 5 | 5 | 5 | 5 | 5 | 5 | 5 | 4 | 2 | 1 | 1 | 3 | 1 | 1 |
| Sensory evaluation | | | | | | | | | | | | | | | | |
| | Freshness | 5 | | | 5 | | | 4 | | | 2 | | | 3 | | |
| | Freedom from stickiness | 5 | | | 5 | | | 5 | | | 1 | | | 3 | | |
| | Body feeling | 5 | | | 5 | | | 5 | | | 2 | | | 3 | | |
| | Extensibility | 5 | | | 5 | | | 5 | | | 3 | | | 2 | | |
| | Smoothness | 4 | | | 5 | | | 5 | | | 2 | | | 3 | | |

[Table 5-1]

| Number | Example 63 | | | Example 64 | | | Example 65 | | | Example 66 | | | Example 67 | | | Example 68 | | | Example 69 | | | Example 70 | | | Example 71 | | | Comparative Example 26 | | | Comparative Example 27 | | |
|---|---|---|---|---|---|---|---|---|---|---|---|---|---|---|---|---|---|---|---|---|---|---|---|---|---|---|---|---|---|---|---|---|---|
| Composition | | | | | | | | | | | | | | | | | | | | | | | | | | | | | | | | | |
| Component A | Yes | | | Yes | | | Yes | | | Yes | | | Yes | | | Yes | | | Yes | | | Yes | | | Yes | | | No | | | Yes | | |
| Component B proportion (%) | 0.032 | | | 1.33 | | | 6.85 | | | 0.032 | | | 1.33 | | | 6.85 | | | 0.032 | | | 1.33 | | | 6.85 | | | 1.33 | | | – | | |
| Component C mass (%) | – | | | – | | | – | | | – | | | – | | | – | | | 1.3 | | | 5.1 | | | 6.5 | | | 5.1 | | | – | | |
| Component D mass (%) | – | | | – | | | – | | | – | | | – | | | – | | | 0.08 | | | 0.14 | | | 2.1 | | | 0.14 | | | – | | |
| Component E mass (%) | – | | | – | | | – | | | 0.009 | | | 0.215 | | | 4.309 | | | 0.009 | | | 0.215 | | | 4.309 | | | 0.215 | | | – | | |
| Cocamide MEA | – | | | – | | | – | | | – | | | – | | | – | | | – | | | – | | | – | | | – | | | – | | |
| Lauramide MEA | – | | | – | | | – | | | – | | | – | | | – | | | – | | | – | | | – | | | – | | | – | | |
| Carbon atoms of R1 to R6 of components A to E | (A,11) (B,11) | | | (A,11) (B,11) | | | (A,11) (B,11) | | | (A,11) (B,11) (E,11) | | | (A,11) (B,11) (E,11) | | | (A,11) (B,11) (E,11) | | | (A,11) (B,11) (C,11) (D,11) (E,11) | | | (A,11) (B,11) (C,11) (D,11) (E,11) | | | (A,11) (B,11) (C,11) (D,11) (E,11) | | | (B,11) (C,11) (D,11) (E,11) | | | (A,11) | | |
| M1 to M7 of components A to D | Na | | | Na | | | Na | | | Na | | | Na | | | Na | | | Na | | | Na | | | Na | | | Na | | | Na | | |
| pH of mixed solution | 5 | | | 5 | | | 5 | | | 5 | | | 5 | | | 5 | | | 5 | | | 5 | | | 5 | | | 5 | | | 5 | | |
| Formulation evaluation | | | | | | | | | | | | | | | | | | | | | | | | | | | | | | | | | |
| pH | 5 | | | 5 | | | 5 | | | 5 | | | 5 | | | 5 | | | 5 | | | 5 | | | 5 | | | 5 | | | 5 | | |
| Emulsification stability confirmation test 1 | After 2.0 months | After 2.5 months | After 3.0 months | After 2.0 months | After 2.5 months | After 3.0 months | After 2.0 months | After 2.5 months | After 3.0 months | After 2.0 months | After 2.5 months | After 3.0 months | After 2.0 months | After 2.5 months | After 3.0 months | After 2.0 months | After 2.5 months | After 3.0 months | After 2.0 months | After 2.5 months | After 3.0 months | After 2.0 months | After 2.5 months | After 3.0 months | After 2.0 months | After 2.5 months | After 3.0 months | After 2.0 months | After 2.5 months | After 3.0 months | After 2.0 months | After 2.5 months | After 3.0 months |
| Ethylhexyl palmitate | 5 | 3 | 2 | 5 | 3 | 2 | 5 | 3 | 3 | 5 | 4 | 3 | 5 | 5 | 4 | 5 | 4 | 4 | 5 | 5 | 5 | 5 | 5 | 5 | 5 | 5 | 4 | 4 | 3 | 1 | 4 | 1 | 1 |
| Olive oil | 5 | 3 | 2 | 5 | 3 | 2 | 5 | 3 | 2 | 5 | 5 | 4 | 5 | 5 | 4 | 5 | 5 | 4 | 5 | 5 | 4 | 5 | 5 | 5 | 5 | 5 | 5 | 2 | 2 | 1 | 5 | 3 | 1 |
| Mineral oil | 5 | 3 | 2 | 5 | 3 | 2 | 5 | 3 | 2 | 5 | 5 | 4 | 5 | 5 | 4 | 5 | 5 | 4 | 5 | 5 | 5 | 5 | 5 | 5 | 5 | 5 | 5 | 4 | 2 | 1 | 4 | 2 | 1 |
| Cyclomethicone | 5 | 3 | 2 | 5 | 3 | 3 | 5 | 3 | 2 | 5 | 5 | 4 | 5 | 5 | 4 | 5 | 5 | 4 | 5 | 5 | 5 | 5 | 5 | 5 | 5 | 5 | 5 | 3 | 1 | 1 | 4 | 2 | 1 |
| Dimethicone | 5 | 3 | 2 | 5 | 3 | 3 | 5 | 3 | 2 | 5 | 4 | 3 | 5 | 5 | 4 | 5 | 4 | 3 | 5 | 5 | 4 | 5 | 5 | 5 | 5 | 5 | 4 | 3 | 2 | 1 | 3 | 2 | 1 |
| Emulsification stability confirmation test 2 | | | | | | | | | | | | | | | | | | | | | | | | | | | | | | | | | |
| Cetostearyl alcohol | 5 | 3 | 2 | 5 | 3 | 2 | 5 | 3 | 2 | 5 | 5 | 4 | 5 | 5 | 4 | 5 | 4 | 3 | 5 | 5 | 5 | 5 | 5 | 5 | 5 | 5 | 5 | 3 | 2 | 1 | 4 | 2 | 1 |
| Myristyl alcohol | 5 | 3 | 3 | 5 | 3 | 2 | 5 | 3 | 2 | 5 | 5 | 4 | 5 | 5 | 4 | 5 | 5 | 4 | 5 | 5 | 5 | 5 | 5 | 5 | 5 | 5 | 5 | 3 | 2 | 1 | 5 | 3 | 1 |
| Dispersion stability confirmation test | After 200 hours | After 224 hours | After 248 hours | After 200 hours | After 224 hours | After 248 hours | After 200 hours | After 224 hours | After 248 hours | After 200 hours | After 224 hours | After 248 hours | After 200 hours | After 224 hours | After 248 hours | After 200 hours | After 224 hours | After 248 hours | After 200 hours | After 224 hours | After 248 hours | After 200 hours | After 224 hours | After 248 hours | After 200 hours | After 224 hours | After 248 hours | After 200 hours | After 224 hours | After 248 hours | After 200 hours | After 224 hours | After 248 hours |
| | 5 | 3 | 2 | 5 | 4 | 3 | 4 | 2 | 1 | 5 | 5 | 2 | 5 | 5 | 4 | 5 | 4 | 2 | 5 | 5 | 5 | 5 | 5 | 5 | 5 | 5 | 4 | 3 | 2 | 1 | 4 | 2 | 1 |
| Sensory evaluation | | | | | | | | | | | | | | | | | | | | | | | | | | | | | | | | | |
| Freshness | 5 | | | 5 | | | 4 | | | 5 | | | 5 | | | 4 | | | 5 | | | 5 | | | 5 | | | 3 | | | 2 | | |
| Freedom from stickiness | 5 | | | 5 | | | 4 | | | 5 | | | 5 | | | 4 | | | 5 | | | 5 | | | 5 | | | 1 | | | 3 | | |
| Body feeling | 3 | | | 3 | | | 3 | | | 3 | | | 3 | | | 3 | | | 5 | | | 5 | | | 5 | | | 2 | | | 2 | | |
| Extensibility | 3 | | | 3 | | | 3 | | | 5 | | | 5 | | | 4 | | | 5 | | | 5 | | | 4 | | | 2 | | | 3 | | |
| Smoothness | 3 | | | 3 | | | 3 | | | 4 | | | 5 | | | 5 | | | 4 | | | 5 | | | 5 | | | 2 | | | 3 | | |

[Table 5-2]

| Number | Example 72 | | | Example 73 | | | Example 74 | | | Example 75 | | | Example 76 | | | Example 77 | | | Example 78 | | | Example 79 | | | Example 80 | | | Comparative Example 28 | | | Comparative Example 29 | | |
|---|---|---|---|---|---|---|---|---|---|---|---|---|---|---|---|---|---|---|---|---|---|---|---|---|---|---|---|---|---|---|---|---|---|
| Composition | | | | | | | | | | | | | | | | | | | | | | | | | | | | | | | | | |
| Component A | Yes | | | Yes | | | Yes | | | Yes | | | Yes | | | Yes | | | Yes | | | Yes | | | Yes | | | No | | | Yes | | |
| Component B proportion (%) | 0.032 | | | 1.33 | | | 6.85 | | | 0.032 | | | 1.33 | | | 6.85 | | | 0.032 | | | 1.33 | | | 6.85 | | | 1.33 | | | – | | |
| Component C mass (%) | – | | | – | | | – | | | – | | | – | | | – | | | 1.3 | | | 5.1 | | | 6.5 | | | 5.1 | | | – | | |
| Component D mass (%) | – | | | – | | | – | | | – | | | – | | | – | | | 0.08 | | | 0.14 | | | 2.1 | | | 0.14 | | | – | | |
| Component E mass (%) | – | | | – | | | – | | | 0.009 | | | 0.215 | | | 4.309 | | | 0.009 | | | 0.215 | | | 4.309 | | | 0.215 | | | – | | |
| Cocamide MEA | – | | | – | | | – | | | – | | | – | | | – | | | – | | | – | | | – | | | – | | | – | | |
| Lauramide MEA | – | | | – | | | – | | | – | | | – | | | – | | | – | | | – | | | – | | | – | | | – | | |
| Carbon atoms of R1 to R6 of components A to E | (A,11) (B,11) | | | (A,11) (B,11) | | | (A,11) (B,11) | | | (A,11) (B,11) (E,11) | | | (A,11) (B,11) (E,11) | | | (A,11) (B,11) (E,11) | | | (A,11) (B,11) (C,11) (D,11) (E,11) | | | (A,11) (B,11) (C,11) (D,11) (E,11) | | | (A,11) (B,11) (C,11) (D,11) (E,11) | | | (B,11) (C,11) (D,11) (E,11) | | | (A,11) | | |
| M1 to M7 of components A to D | Na | | | Na | | | Na | | | Na | | | Na | | | Na | | | Na | | | Na | | | Na | | | Na | | | Na | | |
| pH of mixed solution | 9 | | | 9 | | | 9 | | | 9 | | | 9 | | | 9 | | | 9 | | | 9 | | | 9 | | | 9 | | | 9 | | |
| Formulation evaluation | | | | | | | | | | | | | | | | | | | | | | | | | | | | | | | | | |
| pH | 9 | | | 9 | | | 9 | | | 9 | | | 9 | | | 9 | | | 9 | | | 9 | | | 9 | | | 9 | | | 9 | | |
| Emulsification stability confirmation test 1 | After 2.0 months | After 2.5 months | After 3.0 months | After 2.0 months | After 2.5 months | After 3.0 months | After 2.0 months | After 2.5 months | After 3.0 months | After 2.0 months | After 2.5 months | After 3.0 months | After 2.0 months | After 2.5 months | After 3.0 months | After 2.0 months | After 2.5 months | After 3.0 months | After 2.0 months | After 2.5 months | After 3.0 months | After 2.0 months | After 2.5 months | After 3.0 months | After 2.0 months | After 2.5 months | After 3.0 months | After 2.0 months | After 2.5 months | After 3.0 months | After 2.0 months | After 2.5 months | After 3.0 months |
| Ethylhexyl palmitate | 5 | 3 | 3 | 5 | 3 | 2 | 5 | 3 | 3 | 5 | 4 | 4 | 5 | 5 | 4 | 5 | 4 | 3 | 5 | 5 | 5 | 5 | 5 | 5 | 5 | 5 | 5 | 4 | 3 | 1 | 4 | 2 | 1 |
| Olive oil | 5 | 3 | 3 | 5 | 3 | 2 | 5 | 3 | 3 | 5 | 4 | 3 | 5 | 5 | 5 | 5 | 5 | 4 | 5 | 5 | 4 | 5 | 5 | 5 | 5 | 5 | 5 | 3 | 2 | 1 | 5 | 3 | 1 |
| Mineral oil | 5 | 3 | 3 | 5 | 4 | 3 | 5 | 4 | 3 | 5 | 5 | 4 | 5 | 5 | 4 | 5 | 5 | 4 | 5 | 5 | 5 | 5 | 5 | 5 | 5 | 5 | 5 | 4 | 2 | 1 | 4 | 2 | 1 |
| Cyclomethicone | 5 | 3 | 2 | 5 | 3 | 3 | 5 | 3 | 2 | 5 | 5 | 4 | 5 | 5 | 4 | 5 | 5 | 3 | 5 | 5 | 5 | 5 | 5 | 5 | 5 | 5 | 5 | 3 | 2 | 1 | 4 | 2 | 1 |
| Dimethicone | 5 | 3 | 2 | 5 | 3 | 3 | 5 | 3 | 2 | 5 | 5 | 4 | 5 | 5 | 4 | 5 | 4 | 3 | 5 | 5 | 5 | 5 | 5 | 5 | 5 | 5 | 4 | 2 | 1 | 1 | 5 | 3 | 1 |
| Emulsification stability confirmation test 2 | | | | | | | | | | | | | | | | | | | | | | | | | | | | | | | | | |
| Cetostearyl alcohol | 5 | 3 | 2 | 5 | 4 | 3 | 5 | 3 | 2 | 5 | 5 | 4 | 5 | 5 | 4 | 5 | 5 | 4 | 5 | 5 | 5 | 5 | 5 | 5 | 5 | 5 | 5 | 3 | 2 | 1 | 4 | 2 | 1 |
| Myristyl alcohol | 5 | 3 | 3 | 5 | 3 | 2 | 5 | 3 | 2 | 5 | 5 | 4 | 5 | 5 | 5 | 5 | 5 | 4 | 5 | 5 | 5 | 5 | 5 | 5 | 5 | 5 | 5 | 2 | 1 | 1 | 3 | 1 | 1 |
| Dispersion stability confirmation test | After 200 hours | After 224 hours | After 248 hours | After 200 hours | After 224 hours | After 248 hours | After 200 hours | After 224 hours | After 248 hours | After 200 hours | After 224 hours | After 248 hours | After 200 hours | After 224 hours | After 248 hours | After 200 hours | After 224 hours | After 248 hours | After 200 hours | After 224 hours | After 248 hours | After 200 hours | After 224 hours | After 248 hours | After 200 hours | After 224 hours | After 248 hours | After 200 hours | After 224 hours | After 248 hours | After 200 hours | After 224 hours | After 248 hours |
| | 5 | 4 | 2 | 5 | 4 | 2 | 4 | 2 | 1 | 5 | 5 | 3 | 5 | 5 | 3 | 5 | 5 | 2 | 5 | 5 | 5 | 5 | 5 | 5 | 5 | 5 | 5 | 3 | 2 | 1 | 4 | 2 | 1 |
| Sensory evaluation | | | | | | | | | | | | | | | | | | | | | | | | | | | | | | | | | |
| Freshness | 5 | | | 5 | | | 4 | | | 5 | | | 5 | | | 5 | | | 5 | | | 5 | | | 5 | | | 3 | | | 3 | | |
| Freedom from stickiness | 5 | | | 5 | | | 5 | | | 5 | | | 5 | | | 5 | | | 5 | | | 5 | | | 4 | | | 2 | | | 3 | | |
| Body feeling | 3 | | | 3 | | | 3 | | | 3 | | | 3 | | | 3 | | | 5 | | | 5 | | | 5 | | | 2 | | | 2 | | |
| Extensibility | 3 | | | 3 | | | 3 | | | 5 | | | 5 | | | 4 | | | 5 | | | 5 | | | 5 | | | 3 | | | 3 | | |
| Smoothness | 3 | | | 3 | | | 3 | | | 4 | | | 5 | | | 5 | | | 5 | | | 5 | | | 5 | | | 3 | | | 2 | | |

[Table 5-3]

| Number | Example 81 | | | Example 82 | | | Example 83 | | | Example 84 | | | Example 85 | | | Example 86 | | | Example 87 | | | Example 88 | | | Example 89 | | | Comparative Example 30 | | | Comparative Example 31 | | |
|---|---|---|---|---|---|---|---|---|---|---|---|---|---|---|---|---|---|---|---|---|---|---|---|---|---|---|---|---|---|---|---|---|---|
| Composition | | | | | | | | | | | | | | | | | | | | | | | | | | | | | | | | | |
| Component A | Yes | | | Yes | | | Yes | | | Yes | | | Yes | | | Yes | | | Yes | | | Yes | | | Yes | | | No | | | Yes | | |
| Component B proportion (%) | 0.032 | | | 1.33 | | | 6.85 | | | 0.032 | | | 1.33 | | | 6.85 | | | 0.032 | | | 1.33 | | | 6.85 | | | 1.33 | | | – | | |
| Component C mass (%) | – | | | – | | | – | | | – | | | – | | | – | | | 1.3 | | | 5.1 | | | 6.5 | | | 5.1 | | | – | | |
| Component D mass (%) | – | | | – | | | – | | | – | | | – | | | – | | | 0.08 | | | 0.14 | | | 2.1 | | | 0.14 | | | – | | |
| Component E mass (%) | – | | | – | | | – | | | 0.009 | | | 0.215 | | | 4.309 | | | 0.009 | | | 0.215 | | | 4.309 | | | 0.215 | | | – | | |
| Cocamide MEA | – | | | – | | | – | | | – | | | – | | | – | | | – | | | – | | | – | | | – | | | – | | |
| Lauramide MEA | – | | | – | | | – | | | – | | | – | | | – | | | – | | | – | | | – | | | – | | | – | | |
| Carbon atoms of R1 to R6 of components A to E | (A,11) (B,11) | | | (A,11) (B,11) | | | (A,11) (B,11) | | | (A,11) (B,11) (E,11) | | | (A,11) (B,11) (E,11) | | | (A,11) (B,11) (E,11) | | | (A,11) (B,11) (C,11) (D,11) (E,11) | | | (A,11) (B,11) (C,11) (D,11) (E,11) | | | (A,11) (B,11) (C,11) (D,11) (E,11) | | | (B,11) (C,11) (D,11) (E,11) | | | (A,11) | | |
| M1 to M7 of components A to D | Na | | | Na | | | Na | | | Na | | | Na | | | Na | | | Na | | | Na | | | Na | | | Na | | | Na | | |
| pH of mixed solution | 12 | | | 12 | | | 12 | | | 12 | | | 12 | | | 12 | | | 12 | | | 12 | | | 12 | | | 12 | | | 12 | | |
| Formulation evaluation | | | | | | | | | | | | | | | | | | | | | | | | | | | | | | | | | |
| pH | 12 | | | 12 | | | 12 | | | 12 | | | 12 | | | 12 | | | 12 | | | 12 | | | 12 | | | 12 | | | 12 | | |
| Emulsification stability confirmation test 1 | After 2.0 months | After 2.5 months | After 3.0 months | After 2.0 months | After 2.5 months | After 3.0 months | After 2.0 months | After 2.5 months | After 3.0 months | After 2.0 months | After 2.5 months | After 3.0 months | After 2.0 months | After 2.5 months | After 3.0 months | After 2.0 months | After 2.5 months | After 3.0 months | After 2.0 months | After 2.5 months | After 3.0 months | After 2.0 months | After 2.5 months | After 3.0 months | After 2.0 months | After 2.5 months | After 3.0 months | After 2.0 months | After 2.5 months | After 3.0 months | After 2.0 months | After 2.5 months | After 3.0 months |
| Ethylhexyl palmitate | 5 | 4 | 3 | 5 | 3 | 2 | 5 | 3 | 2 | 5 | 5 | 4 | 5 | 5 | 4 | 5 | 5 | 4 | 5 | 5 | 4 | 5 | 5 | 5 | 5 | 5 | 4 | 3 | 2 | 1 | 4 | 2 | 1 |
| Olive oil | 5 | 3 | 2 | 5 | 3 | 2 | 5 | 2 | 2 | 5 | 4 | 3 | 5 | 5 | 4 | 5 | 5 | 4 | 5 | 5 | 5 | 5 | 5 | 5 | 5 | 5 | 5 | 3 | 2 | 1 | 5 | 3 | 1 |
| Mineral oil | 5 | 3 | 2 | 5 | 3 | 2 | 5 | 3 | 2 | 5 | 5 | 4 | 5 | 5 | 4 | 5 | 5 | 4 | 5 | 5 | 5 | 5 | 5 | 5 | 5 | 5 | 5 | 3 | 2 | 1 | 4 | 2 | 1 |
| Cyclomethicone | 5 | 4 | 3 | 5 | 4 | 3 | 5 | 3 | 2 | 5 | 5 | 4 | 5 | 5 | 4 | 5 | 4 | 3 | 5 | 5 | 5 | 5 | 5 | 5 | 5 | 5 | 4 | 3 | 2 | 1 | 4 | 2 | 1 |
| Dimethicone | 5 | 3 | 2 | 5 | 4 | 3 | 5 | 3 | 2 | 5 | 4 | 3 | 5 | 5 | 4 | 5 | 5 | 3 | 5 | 5 | 4 | 5 | 5 | 5 | 5 | 5 | 5 | 2 | 1 | 1 | 4 | 2 | 1 |
| Emulsification stability confirmation test 2 | | | | | | | | | | | | | | | | | | | | | | | | | | | | | | | | | |
| Cetostearyl alcohol | 5 | 4 | 3 | 5 | 3 | 2 | 5 | 3 | 2 | 5 | 5 | 4 | 5 | 5 | 4 | 5 | 4 | 3 | 5 | 5 | 5 | 5 | 5 | 5 | 5 | 5 | 5 | 4 | 3 | 1 | 3 | 1 | 1 |
| Myristyl alcohol | 5 | 3 | 3 | 5 | 3 | 2 | 5 | 3 | 2 | 5 | 5 | 4 | 5 | 5 | 4 | 5 | 5 | 4 | 5 | 5 | 5 | 5 | 5 | 5 | 5 | 5 | 4 | 3 | 2 | 1 | 3 | 1 | 1 |
| Dispersion stability confirmation test | After 200 hours | After 224 hours | After 248 hours | After 200 hours | After 224 hours | After 248 hours | After 200 hours | After 224 hours | After 248 hours | After 200 hours | After 224 hours | After 248 hours | After 200 hours | After 224 hours | After 248 hours | After 200 hours | After 224 hours | After 248 hours | After 200 hours | After 224 hours | After 248 hours | After 200 hours | After 224 hours | After 248 hours | After 200 hours | After 224 hours | After 248 hours | After 200 hours | After 224 hours | After 248 hours | After 200 hours | After 224 hours | After 248 hours |
| | 5 | 4 | 2 | 5 | 4 | 3 | 4 | 2 | 1 | 5 | 5 | 3 | 5 | 5 | 3 | 5 | 4 | 3 | 5 | 5 | 4 | 5 | 5 | 5 | 5 | 5 | 4 | 3 | 2 | 1 | 4 | 1 | 1 |
| Sensory evaluation | | | | | | | | | | | | | | | | | | | | | | | | | | | | | | | | | |
| Freshness | 5 | | | 5 | | | 4 | | | 5 | | | 5 | | | 5 | | | 5 | | | 5 | | | 5 | | | 3 | | | 3 | | |
| Freedom from stickiness | 5 | | | 5 | | | 5 | | | 5 | | | 5 | | | 4 | | | 5 | | | 5 | | | 5 | | | 3 | | | 3 | | |
| Body feeling | 3 | | | 3 | | | 3 | | | 3 | | | 3 | | | 3 | | | 5 | | | 5 | | | 5 | | | 2 | | | 2 | | |
| Extensibility | 3 | | | 3 | | | 3 | | | 5 | | | 5 | | | 5 | | | 5 | | | 5 | | | 4 | | | 3 | | | 2 | | |
| Smoothness | 3 | | | 3 | | | 3 | | | 5 | | | 5 | | | 5 | | | 4 | | | 5 | | | 5 | | | 2 | | | 3 | | |

The compositions of Examples, as containing at least the components A and B, the components A, C, and D, or the components A and E, were evaluated good for all of the emulsification stability, dispersion stability, and feeling in use. In contrast, Comparative Examples, which are compositions not corresponding to the invention, were inferior in both the emulsification stability and dispersion stability to those of Examples.

Industrial Applicability

According to the starting material composition for fragrances and cosmetics according to the present invention, excellent formulation for fragrances and cosmetics can be provided, which formulation has not only initial emulsification performance and dispersion performance but also good long-term emulsification stability and dispersibility, and the like.

## Claims

**1.** A composition comprising:

a component A shown below; and
one or more components selected from the group consisting of a component B, a component C, a component D, and a component E each shown below, provided that only the component C or only the component D is excluded:

• component A: an acyl group-containing amino acid derivative represented by the following general formula (A) or a salt thereof:

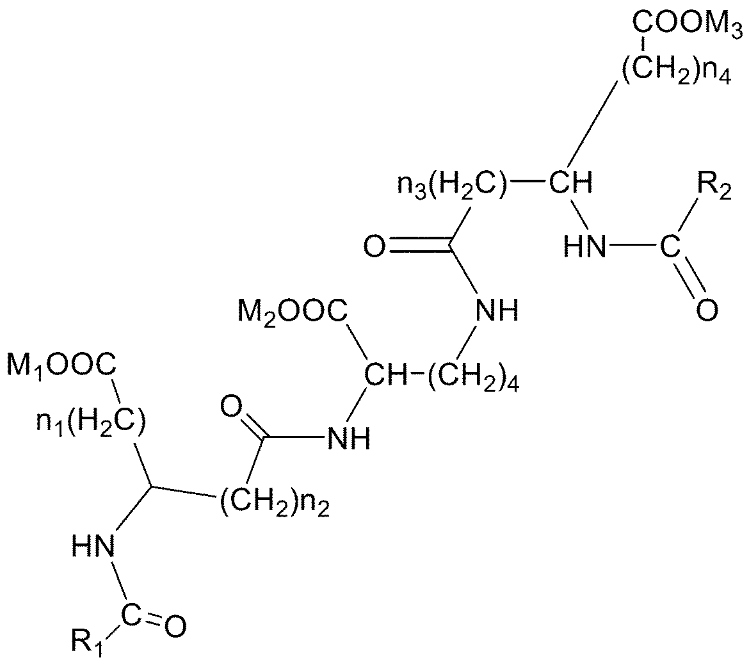

(A)

wherein

R1 and R2 are each independently a saturated or unsaturated linear or branched hydrocarbon group,
M1, M2, and M3 are each independently a hydrogen atom, alkali metal, alkaline earth metal, an organic or inorganic amine, a basic amino acid, choline, aluminum, or zinc,
n1 and n2 are 0 and 2 or 2 and 0, and
n3 and n4 are 0 and 2 or 2 and 0,

• component B: a N-acyl pyrrolidonecarboxylic acid represented by the following general formula (B) or a salt thereof:

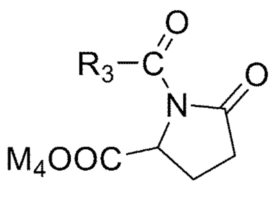

(B)

wherein

R3 is a saturated or unsaturated linear or branched hydrocarbon group, and
M4 is a hydrogen atom, alkali metal, alkaline earth metal, an organic or inorganic amine, a basic amino acid, choline, aluminum, or zinc,

• component C: an acyl glutamic acid represented by the following general formula (C) or salt thereof:

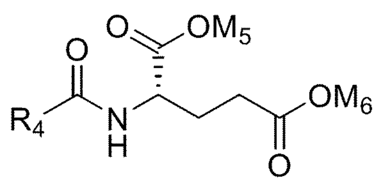

(C)

wherein

R4 is a saturated or unsaturated linear or branched hydrocarbon group, and
M5 and M6 are each independently a hydrogen atom, alkali metal, alkaline earth metal, an organic or inorganic amine, a basic amino acid, choline, aluminum, or zinc,

• component D: a fatty acid represented by the following general formula (D) or salt thereof:

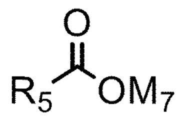 (D)

wherein

R5 is a saturated or unsaturated linear or branched hydrocarbon group, and
M7 is a hydrogen atom, alkali metal, alkaline earth metal, an organic or inorganic amine, a basic amino acid, choline, aluminum, or zinc, and

• component E: a fatty acid amide represented by the following general formula (E):

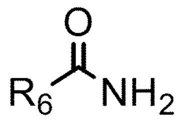 (E)

wherein
R6 is a saturated or unsaturated linear or branched hydrocarbon group.

**2.** The composition according to claim 1, comprising the component A and the component B.

**3.** The composition according to claim 1, comprising the component A and the component E.

**4.** The composition according to claim 1, comprising the component A, the component B, and the component E.

**5.** The composition according to any of claims 1 to 4, wherein R1 to R6 in the general formulas (A) to (E) are each independently a hydrocarbon group having 1 to 29 carbon atoms.

**6.** The composition according to any of claims 1 to 5, wherein M1 to M7 in the general formulas (A) to (D) are each independently Na, K, triethanolamine, Mg, Ca, Al, Zn, arginine, or H.

**7.** The composition according to any of claims 1, 2, 4, 5, and 6, wherein a proportion of the component B with respect to the component A is 0.004% or more.

**8.** The composition according to any of claims 1 and 5 to 7, wherein

an amount of the component C is 0.8% by mass or more based on the total mass of the component A and the component C, and
an amount of the component D is 0.06% by mass or more based on the total mass of the component A and the component D.

**9.** The composition according to any of claims 1 and 3 to 8, wherein an amount of the component E is 0.005% by mass or more based on the mass of the component A.

**10.** A method for producing the composition according to any of claims 1, 2, and 4 to 9, comprising the following steps:

1) reacting glutamic acid or a salt thereof with a fatty acid chloride in a mixed solvent comprising water and an organic solvent to provide a reaction liquid containing a N-acyl glutamic acid or a salt thereof, allowing the

reaction liquid to have a pH of 1 to 6 with an acid, and separating the reaction liquid into an organic layer and an aqueous layer at a temperature of 25 to 80°C;
2) concentrating the organic layer to dryness under acidity to convert a portion of the N-acyl glutamic acid or a salt thereof into a N-acyl pyrrolidonecarboxylic acid or a salt thereof;
3) reacting the N-acyl glutamic acid or a salt thereof with an acid anhydride to provide a N-acyl glutamic acid anhydride; and
4) reacting the N-acyl glutamic acid anhydride with lysine or a salt thereof to provide an acyl group-containing amino acid derivative or a salt thereof.

**11.** The production method according to claim 10, wherein, in the step 2), the organic layer is concentrated to dryness until a loss on drying reaches 10% by mass or less.

**12.** The production method according to claim 10 or 11, wherein the concentration to dryness in the step 2) is performed under reduced pressure at 50°C or more.

**13.** The composition according to any of claims 1 to 9, wherein the composition is a starting material composition for fragrances and cosmetics.

**14.** Use of the composition according to any of claims 1 to 9 as a starting material for fragrances and cosmetics.

**15.** A cosmetic agent comprising the composition according to any of claims 1 to 9.

**16.** The cosmetic agent according to claim 15, having a pH of 4 or more.

## INTERNATIONAL SEARCH REPORT

International application No. **PCT/JP2021/039903**

### A. CLASSIFICATION OF SUBJECT MATTER

***C09K 23/00***(2022.01)i; ***C09K 23/22***(2022.01)i; ***C09K 23/28***(2022.01)i; ***C09K 23/32***(2022.01)i; ***A61Q 19/00***(2006.01)i; ***A61Q 5/00***(2006.01)i; ***A61K 8/04***(2006.01)i; ***A61K 8/06***(2006.01)i; ***A61K 8/36***(2006.01)i; ***A61K 8/42***(2006.01)i; ***A61K 8/44***(2006.01)i; ***A61K 8/49***(2006.01)i; ***A61K 8/64***(2006.01)i
FI: B01F17/28; A61K8/49; A61Q19/00; A61Q5/00; A61K8/06; A61K8/04; A61K8/36; A61K8/42; A61K8/44; B01F17/00; B01F17/22; B01F17/32; A61K8/64

According to International Patent Classification (IPC) or to both national classification and IPC

### B. FIELDS SEARCHED

Minimum documentation searched (classification system followed by classification symbols)

B01F17/00; B01F17/22; B01F17/28; B01F17/32; A61Q19/00; A61Q5/00; A61K8/04; A61K8/06; A61K8/36; A61K8/42; A61K8/44; A61K8/49; A61K8/64

Documentation searched other than minimum documentation to the extent that such documents are included in the fields searched

Published examined utility model applications of Japan 1922-1996
Published unexamined utility model applications of Japan 1971-2021
Registered utility model specifications of Japan 1996-2021
Published registered utility model applications of Japan 1994-2021

Electronic data base consulted during the international search (name of data base and, where practicable, search terms used)

JSTPlus/JMEDPlus/JST7580 (JDreamIII); CAplus/REGISTRY (STN)

### C. DOCUMENTS CONSIDERED TO BE RELEVANT

| Category* | Citation of document, with indication, where appropriate, of the relevant passages | Relevant to claim No. |
|---|---|---|
| X<br>A | JP 2020-164448 A (KOSE CORP) 08 October 2020 (2020-10-08)<br>examples 23, paragraph [0069] | 1, 5-6, 8, 13-16<br>10-12 |
| X<br>A | JP 2011-39229 A (FUJI XEROX CO LTD) 24 February 2011 (2011-02-24)<br>claims, paragraphs [0029]-[0030], examples 1-7 | 1, 3, 5-6, 9<br>10-12 |
| Y<br>A | JP 2018-43971 A (OSHIRO, Fumitoshi) 22 March 2018 (2018-03-22)<br>claims, paragraph [0051], examples 1-10 | 1, 3-6, 9, 13-16<br>10-12 |
| Y | JP 2020-121935 A (SHISEIDO CO LTD) 13 August 2020 (2020-08-13)<br>paragraph [0057] | 1, 3-6, 9, 13-16 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **23 December 2021** | **11 January 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | <br><br>Telephone No. |

Form PCT/ISA/210 (second sheet) (January 2015)

**INTERNATIONAL SEARCH REPORT**

International application No.
**PCT/JP2021/039903**

**C. DOCUMENTS CONSIDERED TO BE RELEVANT**

| Category* | Citation of document, with indication, where appropriate, of the relevant passages | Relevant to claim No. |
|---|---|---|
| Y | JP 2020-125374 A (KOKYU ALCOHOL KOGYO CO LTD) 20 August 2020 (2020-08-20) claims, paragraph [0026] | 1, 3-6, 9, 13-16 |
| Y | JP 2010-105964 A (ADERANS CO LTD) 13 May 2010 (2010-05-13) claims, examples, comparative examples 1-2, 4, paragraph [0045] | 1-2, 5-7, 13-16 |
| Y | JP 2019-99536 A (TOSAKA, Masako) 24 June 2019 (2019-06-24) paragraphs [0001], [0016], examples 1-3 | 1-2, 5-7, 13-16 |
| Y | JP 2016-53093 A (L'OREAL) 14 April 2016 (2016-04-14) claims, paragraph [0153] | 1-2, 4-7, 13-16 |

Form PCT/ISA/210 (second sheet) (January 2015)

**INTERNATIONAL SEARCH REPORT**
**Information on patent family members**

International application No.
**PCT/JP2021/039903**

| Patent document cited in search report | Publication date (day/month/year) | Patent family member(s) | Publication date (day/month/year) |
|---|---|---|---|
| JP 2020-164448 A | 08 October 2020 | (Family: none) | |
| JP 2011-39229 A | 24 February 2011 | (Family: none) | |
| JP 2018-43971 A | 22 March 2018 | (Family: none) | |
| JP 2020-121935 A | 13 August 2020 | WO 2020/158667 A1 | |
| JP 2020-125374 A | 20 August 2020 | (Family: none) | |
| JP 2010-105964 A | 13 May 2010 | (Family: none) | |
| JP 2019-99536 A | 24 June 2019 | (Family: none) | |
| JP 2016-53093 A | 14 April 2016 | US 2011/0142897 A1<br>claims, paragraph [0177]<br>EP 2335683 A1<br>CN 102106642 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 4070768 B
- JP 2006160686 A